# EUROPEAN PATENT APPLICATION

(11) **EP 4 844 059 A1**
(43) Date of publication of application: **25.11.2026**
(21) Application number: 26178091.0
(22) Date of filing: 12.05.2026
(51) Int. Cl.: C12N 9/02

(54) **METHOD OF ENZYMATIC C19 HYDROXYLATION OF A STEROID**

(30) Priority: 19.05.2025 EP 25177305
(71) Applicant: Bayer Aktiengesellschaft, 51373 Leverkusen (DE)
(72) Inventor: Wolf, Timo, 51373 Leverkusen (DE); Basso, Fabian, 51373 Leverkusen (DE); Kensch, Oliver, 51373 Leverkusen (DE); Richter, Florian, 51373 Leverkusen (DE); Ludwig, Marcus, 51373 Leverkusen (DE); Sassi, Jamaleddine, 51373 Leverkusen (DE); Heyman, Benedikt, 51373 Leverkusen (DE); Gremm, Lisa, 51373 Leverkusen (DE); Thede, Kai, 13353 Berlin (DE); Wagner, Jakob, 13353 Berlin (DE)
(74) Representative: BIP Patents

(57) **Abstract**

What is described herein relates to a Cytochrome P450 BM3 monooxygenase (BM3) variant for catalyzing the C19 hydroxylation of a steroid or steroid derivative, wherein the BM3 variant comprises compared to SEQ ID NO 1 at least the mutations D80I and H236I and K440C.

## Description

### FIELD OF THE DISCLOSURE

The present invention relates a novel in vitro method for enzymatic C19 hydroxylation of a steroid. In particular, the present invention also relates to in vitro methods and processes using P450-BM3 variants for the production of estrone.

### BACKGROUND

The steroid hormones estrone and estradiol and their derivatives are used as medications, for instance in menopausal hormone therapy, contraception and oncology or are important intermediates for the synthesis of further steroid products. Estrone and estradiol can be generated in several steps starting from phytosterols. The different degradation products of phytosterols contain a methyl group in position C19 of the steroid ring systems which needs to be removed in order to synthesize estrone and estradiol and their derivatives under aromatization of the A ring of the steroid ring system. Inhoffen describes this aromatization by thermolysis for the synthesis of estradiol (Inhoffen 1947) and Hershberg *et al* describe this aromatization by thermolysis for the synthesis of estrone (Hershberg 1950). However, yields are not optimal and various side products are formed. WO2021156200 A1 describes P450 BM3 variants for the selective hydroxylation at the C19 position of the steroid ring system for example it was found that C19-hydroxylation of substrates testosterone, delta-1-testosterone and androsta-1,4-dien-3,17-dione (ADD) can be achieved using mutants of bacterial cytochrome P450 BM3 monooxygenases as biocatalysts.

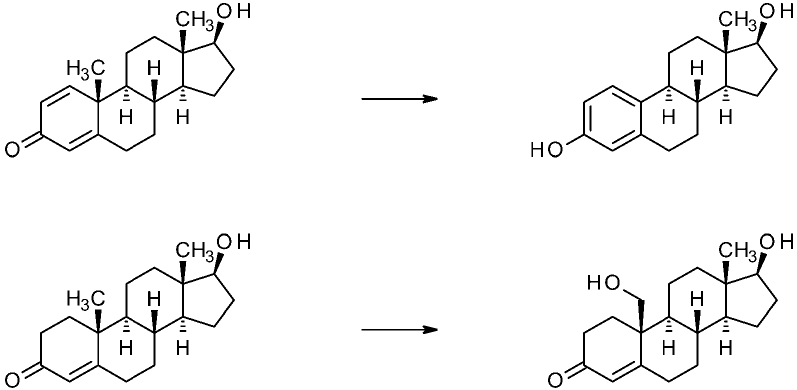

However, the space time yield of C19 hydroxylation was very low.

According to what is described herein it was now nevertheless surprisingly found that via employing novel P450 BM3 variants the space time yield of C19 hydroxylation could be markedly improved.

### DETAILED DESCRIPTION

### BRIEF DESCRIPTION OF THE SEQUENCE IDs

The Sequence Listing associated with this application is filed in electronic format and hereby incorporated by reference into the specification in its entirety.

**Table: 1**

| SEQ ID NO | AA Sequence | Mutations compared to SEQ ID NO 1 |
|---|---|---|
| 1 | AIKEMPQPKTFGELKNLPLLNTDKPVQALMKIADELGEIFKFEAPGRVTRYL SSQRLIKEACDESRFDKNLGQALKFLRDFEGDGLATSWTHEKNWKKAHNI LLPSFSQQAMKGYHAMMVDIAVQLVQKWERLNADEHIEVPEDMTRLTLD TIGLCGFNYRFNSFYRDQPHPFITSYPRAYDEYMNKFQRANPDDPAYDENK RQFQEDIKVMNDLVDKIIADRKASGEQSDDLLTHMLNGKDPETGEPLDDE NIRYQIITFLIAGHETTSGLLSFALYFLVKNPHVLQKAAEEAARVLVDPVPS YKQVKQLKYVGMVLNEALRLWPTAPAFSLYAKEDTVLGGEYPLEKGDEL MVLIPQLHRDKTIWGDDVEEFRPERFENPSAIPQHAFKPFGNGQRACIGQQF ALHEATLVLGMMLKHFDFEDHTNYELDIKETLTLKPEGFVVKAKSKKIPLG GIPSPSTEQSAKKVRKKAENAHNTPLLVLYGSNMGTAEGTARDLADIAMS KGFAPQVATLDSHAGNLPREGAVLIVTASYNGHPPDNAKQFVDWLDQASA DEVKGVRYSVFGCGDKNWATTYQKVPAFIDETLAAKGAENIADRGEADA SDDFEGTYEEWREHMWSDVAAYFNLDIENSEDNKSTLSLQFVDSAADMPL AKMHGAFSTNVVASKELQQPGSARSTRHLEIELPKEASYQEGDHLGVIPRN YEGIVNRVTARFGLDASQQIRLEAEEEKLAHLPLAKTVSVEELLQYVELQD PVTRTQLRAMAAKTVCPPHKVELEALLEKQAYKEQVLAKRLTMLELLEKY PACEMKFSEFIALLPSIRPRYYSISSSPRVDEKQASITVSVVSGEAWSGYGEY KGIASNYLAELQEGDTITCFISTPQSEFTLPKDPETPLIMVGPGTGVAPFRGF VQARKQLKEQGQSLGEAHLYFGCRSPHEDYLYQEELENAQSEGIITLHTAF SRMPNQPKTYVQHVMEQDGKKLIELLDQGAHFYICGDGSQMAPAVEATL MKSYADVHQVSEADARLWLQQLEEKGRYAKDVWAG | |
| 2 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKVSGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, D80I, T175Y, A225V, H236I, G240R, K440C |
| 3 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKVSGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL | Q73W, D80I, T175Y, A225V, H236I, K440C |
| | HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | |
| 4 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYHEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, D80I, T175Y, D182H, H236I, G240R, K440C |
| 5 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYHEYMNKFQRANPDDPAYDENKR QFQEDIKVMNDLVDKIIADRKVSGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, D80I, T175Y, D182H, A225V, H236I, G240R, K440C |
| 6 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYHEYMNKFQRANPDDPAYDENKR QFQEDIKVMNDLVDKIIADRKVSGEQSDDLLTIMLNGKDPETGEPLDDENI RYQIITFLIAGHETTSGLLSFALYFLVKNPHVLQKAAEEAARVLVDPVPSYK | Q73W, D80I, T175Y, D182H, A225V, H236I, K440C |
| | QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | |
| 7 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYHEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | Q73W, D80I, T175Y, D182H, H236I, K440C |
| 8 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYIEYMNKFQRANPDDPAYDENKRQ FQEDIKVMNDLVDKIIADRKASGEQSDDLLTIMLNRKDPETGEPLDDENIRY QIITFLIAGHETTSGLLSFALYFLVKNPHVLQKAAEEAARVLVDPVPSYKQV KQLKYVGMVLNEALRLWPTAPAFSLYAKEDTVLGGEYPLEKGDELMVLIP QLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, D80I, T175Y, D182I, H236I, G240R, K440C |
| 9 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYIEYMNKFQRANPDDPAYDENKRQ | Q73W, D80I, T175Y, D182I, A225V, H236I, G240R, K440C |
| | FQEDIKVMNDLVDKIIADRKVSGEQSDDLLTIMLNRKDPETGEPLDDENIRY QIITFLIAGHETTSGLLSFALYFLVKNPHVLQKAAEEAARVLVDPVPSYKQV KQLKYVGMVLNEALRLWPTAPAFSLYAKEDTVLGGEYPLEKGDELMVLIP QLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 10 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYIEYMNKFQRANPDDPAYDENKRQ FQEDIKVMNDLVDKIIADRKVSGEQSDDLLTIMLNGKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, D80I, T175Y, D182I, A225V, H236I, K440C |
| 11 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYIEYMNKFQRANPDDPAYDENKRQ FQEDIKVMNDLVDKIIADRKASGEQSDDLLTIMLNGKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, D80I, T175Y, D182I, H236I, K440C |
| 12 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL | Q73W, K76I, D80I, T175Y, H236I, G240R, K440C |
| | LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 13 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKVSGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, K76I, D80I, T175Y, A225V, H236I, G240R, K440C |
| 14 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKVSGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | Q73W, K76I, D80I, T175Y, A225V, H236I, K440C |
| 15 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYHEYMNKFQRANPDDPAYDENKR QFQEDIKVMNDLVDKIIADRKVSGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, K76I, D80I, T175Y, D182H, A225V, H236I, G240R, K440C |
| 16 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYIEYMNKFQRANPDDPAYDENKRQ FQEDIKVMNDLVDKIIADRKASGEQSDDLLTIMLNRKDPETGEPLDDENIRY QIITFLIAGHETTSGLLSFALYFLVKNPHVLQKAAEEAARVLVDPVPSYKQV KQLKYVGMVLNEALRLWPTAPAFSLYAKEDTVLGGEYPLEKGDELMVLIP QLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, K76I, D80I, T175Y, D182I, H236I, G240R, K440C |
| 17 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYIEYMNKFQRANPDDPAYDENKRQ FQEDIKVMNDLVDKIIADRKVSGEQSDDLLTIMLNRKDPETGEPLDDENIRY QIITFLIAGHETTSGLLSFALYFLVKNPHVLQKAAEEAARVLVDPVPSYKQV KQLKYVGMVLNEALRLWPTAPAFSLYAKEDTVLGGEYPLEKGDELMVLIP QLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP | Q73W, K76I, D80I, T175Y, D182I, A225V, H236I, G240R, K440C |
| | NQPKTYVQHVMEQDGKKLIELLDQGAHFYICGDGSQMAPAVEATLMKSY ADVHQVSEADARLWLQQLEEKGRYAKDVWAG | |
| 18 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYDEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, K76I, D80I, T175Y, R179L, H236I, G240R, K440C |
| 19 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYIEYMNKFQRANPDDPAYDENKRQ FQEDIKVMNDLVDKIIADRKASGEQSDDLLTIMLNRKDPETGEPLDDENIRY QIITFLIAGHETTSGLLSFALYFLVKNPHVLQKAAEEAARVLVDPVPSYKQV KQLKYVGMVLNEALRLWPTAPAFSLYAKEDTVLGGEYPLEKGDELMVLIP QLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, K76I, D80I, T175Y, R179L, D182I, H236I, G240R, K440C |
| 20 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG | Q73W, K76I, D80I, T175Y, H236I, K440C |
| | IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | |
| 21 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYDEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, D80I, T175Y, R179L, H236I, G240R, K440C |
| 22 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYDEYMNKFQRANPDDPAYDENKR QFQEDIKVMNDLVDKIIADRKVSGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, D80I, T175Y, R179L, A225V, H236I, G240R, K440C |
| 23 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYHEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT | Q73W, D80I, T175Y, R179L, D182H, H236I, G240R, K440C |
| | QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 24 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYHEYMNKFQRANPDDPAYDENKR QFQEDIKVMNDLVDKIIADRKVSGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, D80I, T175Y, R179L, D182H, A225V, H236I, G240R, K440C |
| 25 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYHEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | Q73W, D80I, T175Y, R179L, D182H, H236I, K440C |
| 26 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYIEYMNKFQRANPDDPAYDENKRQ FQEDIKVMNDLVDKIIADRKASGEQSDDLLTIMLNRKDPETGEPLDDENIRY QIITFLIAGHETTSGLLSFALYFLVKNPHVLQKAAEEAARVLVDPVPSYKQV KQLKYVGMVLNEALRLWPTAPAFSLYAKEDTVLGGEYPLEKGDELMVLIP QLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH | Q73W, D80I, T175Y, R179L, D182I, H236I, G240R, K440C |
| | GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 27 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYIEYMNKFQRANPDDPAYDENKRQ FQEDIKVMNDLVDKIIADRKVSGEQSDDLLTIMLNRKDPETGEPLDDENIRY QIITFLIAGHETTSGLLSFALYFLVKNPHVLQKAAEEAARVLVDPVPSYKQV KQLKYVGMVLNEALRLWPTAPAFSLYAKEDTVLGGEYPLEKGDELMVLIP QLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, D80I, T175Y, R179L, D182I, A225V, H236I, G240R, K440C |
| 28 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYIEYMNKFQRANPDDPAYDENKRQ FQEDIKVMNDLVDKIIADRKVSGEQSDDLLTIMLNGKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, D80I, T175Y, R179L, D182I, A225V, H236I, K440C |
| 29 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYIEYMNKFQRANPDDPAYDENKRQ FQEDIKVMNDLVDKIIADRKASGEQSDDLLTIMLNGKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK | Q73W, D80I, T175Y, R179L, D182I, H236I, K440C |
| | GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 30 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYDEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | Q73W, D80I, T175Y, R179L, H236I, K440C |
| 31 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | Q73W, D80I, T175Y, H236I, K440C |
| 32 | AIKEMPQPKTFGELKNLPLLNTDKPVQALMKIADELGEIFKFEAPGRVTRYL SSQRLIKEACDESRFDKNLGLALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP | Q73L, D80I, T175Y, H236I, G240R, K440C |
| | STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 33 | AIKEMPQPKTFGELKNLPLLNTDKPVQALMKIADELGEIFKFEAPGRVTRYL SSQRLIKEACDESRFDKNLGLALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKVSGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73L, D80I, T175Y, A225V, H236I, G240R, K440C |
| 34 | AIKEMPQPKTFGELKNLPLLNTDKPVQALMKIADELGEIFKFEAPGRVTRYL SSQRLIKEACDESRFDKNLGLALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKVSGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | Q73L, D80I, T175Y, A225V, H236I, K440C |
| 35 | AIKEMPQPKTFGELKNLPLLNTDKPVQALMKIADELGEIFKFEAPGRVTRYL SSQRLIKEACDESRFDKNLGLALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYHEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI | Q73L, D80I, T175Y, D182H, H236I, G240R, K440C |
| | PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 36 | AIKEMPQPKTFGELKNLPLLNTDKPVQALMKIADELGEIFKFEAPGRVTRYL SSQRLIKEACDESRFDKNLGLALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYIEYMNKFQRANPDDPAYDENKRQ FQEDIKVMNDLVDKIIADRKASGEQSDDLLTIMLNRKDPETGEPLDDENIRY QIITFLIAGHETTSGLLSFALYFLVKNPHVLQKAAEEAARVLVDPVPSYKQV KQLKYVGMVLNEALRLWPTAPAFSLYAKEDTVLGGEYPLEKGDELMVLIP QLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73L, D80I, T175Y, D182I, H236I, G240R, K440C |
| 37 | AIKEMPQPKTFGELKNLPLLNTDKPVQALMKIADELGEIFKFEAPGRVTRYL SSQRLIKEACDESRFDKNLGLALIFLRIFEGDGLATSWTHEKNWKKAHNILL PSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73L, K76I, D80I, T175Y, H236I, G240R, K440C |
| 38 | AIKEMPQPKTFGELKNLPLLNTDKPVQALMKIADELGEIFKFEAPGRVTRYL SSQRLIKEACDESRFDKNLGLALIFLRIFEGDGLATSWTHEKNWKKAHNILL PSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKVSGEQSDDLLTIMLNRKDPETGEPLDDENIR | Q73L, K76I, D80I, T175Y, A225V, H236I, G240R, K440C |
| | YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 39 | AIKEMPQPKTFGELKNLPLLNTDKPVQALMKIADELGEIFKFEAPGRVTRYL SSQRLIKEACDESRFDKNLGLALIFLRIFEGDGLATSWTHEKNWKKAHNILL PSFSQQAMKGYHAMMVDIAVQLVQKWERLNADEHIEVPEDMTRLTLDTI GLCGFNYRFNSFYRDQPHPFIYSYPLAYDEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73L, K76I, D80I, T175Y, R179L, H236I, G240R, K440C |
| 40 | AIKEMPQPKTFGELKNLPLLNTDKPVQALMKIADELGEIFKFEAPGRVTRYL SSQRLIKEACDESRFDKNLGLALIFLRIFEGDGLATSWTHEKNWKKAHNILL PSFSQQAMKGYHAMMVDIAVQLVQKWERLNADEHIEVPEDMTRLTLDTI GLCGFNYRFNSFYRDQPHPFIYSYPLAYDEYMNKFQRANPDDPAYDENKR QFQEDIKVMNDLVDKIIADRKVSGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73L, K76I, D80I, T175Y, R179L, A225V, H236I, G240R, K440C |
| 41 | AIKEMPQPKTFGELKNLPLLNTDKPVQALMKIADELGEIFKFEAPGRVTRYL SSQRLIKEACDESRFDKNLGLALIFLRIFEGDGLATSWTHEKNWKKAHNILL PSFSQQAMKGYHAMMVDIAVQLVQKWERLNADEHIEVPEDMTRLTLDTI | Q73L, K76I, D80I, T175Y, R179L, D182H, A225V, H236I, G240R, K440C |
| | GLCGFNYRFNSFYRDQPHPFIYSYPLAYHEYMNKFQRANPDDPAYDENKR QFQEDIKVMNDLVDKIIADRKVSGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 42 | AIKEMPQPKTFGELKNLPLLNTDKPVQALMKIADELGEIFKFEAPGRVTRYL SSQRLIKEACDESRFDKNLGLALIFLRIFEGDGLATSWTHEKNWKKAHNILL PSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | Q73L, K76I, D80I, T175Y, H236I, K440C |
| 43 | AIKEMPQPKTFGELKNLPLLNTDKPVQALMKIADELGEIFKFEAPGRVTRYL SSQRLIKEACDESRFDKNLGLALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYDEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73L, D80I, T175Y, R179L, H236I, G240R, K440C |
| 44 | AIKEMPQPKTFGELKNLPLLNTDKPVQALMKIADELGEIFKFEAPGRVTRYL SSQRLIKEACDESRFDKNLGLALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYDEYMNKFQRANPDDPAYDENKR QFQEDIKVMNDLVDKIIADRKVSGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | Q73L, D80I, T175Y, R179L, A225V, H236I, K440C |
| 45 | AIKEMPQPKTFGELKNLPLLNTDKPVQALMKIADELGEIFKFEAPGRVTRYL SSQRLIKEACDESRFDKNLGLALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYIEYMNKFQRANPDDPAYDENKRQ FQEDIKVMNDLVDKIIADRKVSGEQSDDLLTIMLNGKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73L, D80I, T175Y, R179L, D182I, A225V, H236I, K440C |
| 46 | AIKEMPQPKTFGELKNLPLLNTDKPVQALMKIADELGEIFKFEAPGRVTRYL SSQRLIKEACDESRFDKNLGLALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYIEYMNKFQRANPDDPAYDENKRQ FQEDIKVMNDLVDKIIADRKASGEQSDDLLTIMLNGKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP | Q73L, D80I, T175Y, R179L, D182I, H236I, K440C |
| | NQPKTYVQHVMEQDGKKLIELLDQGAHFYICGDGSQMAPAVEATLMKSY ADVHQVSEADARLWLQQLEEKGRYAKDVWAG | |
| 47 | AIKEMPQPKTFGELKNLPLLNTDKPVQALMKIADELGEIFKFEAPGRVTRYL SSQRLIKEACDESRFDKNLGLALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYDEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | Q73L, D80I, T175Y, R179L, H236I, K440C |
| 48 | AIKEMPQPKTFGELKNLPLLNTDKPVQALMKIADELGEIFKFEAPGRVTRYL SSQRLIKEACDESRFDKNLGLALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | Q73L, D80I, T175Y, H236I, K440C |
| 49 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPWITSYPRAYDEYMNKFQRANPDDPAYDENKR QFQEDIKVMNDLVDKIIADRKASGEQSDDLLTIMLNRKDPETGEPLDDENIR YQIITFLIAGHETVSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA | D80I, F173W, H236I, G240R, T269V, K440C |
| | SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 50 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPWIYSYPRAYIEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | D80I, F173W, T175Y, D182I, H236I, K440C |
| 51 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPWIYSYPRAYDEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | D80I, F173W, T175Y, H236I, K440C |
| 52 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPWIYSYPLAYHEYMNKFQRANPDDPAYDENKR QFQEDIKVMNDLVDKIIADRKASGEQSDDLLTIMLNGKDPETGEPLDDENI RYQIITFLIAGHETVSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV | D80I, F173W, T175Y, R179L, D182H, H236I, T269V, K440C |
| | TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | |
| 53 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFITSYPRAYDEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | D80I, H236I, K440C |
| 54 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFITSYPRAYDEYMNKFQRANPDDPAYDENKR QFQEDIKVMNDLVDKIIADRKASGEQSDDLLTIMLNGKDPETGEPLDDENI RYQIITFLIAGHETVSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | D80I, H236I, T269V, K440C |
| 55 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHGWITSYPLAYDEYMNKFQRANPDDPAYDENKR QFQEDIKVMNDLVDKIIADRKASGEQSDDLLTIMLNRKDPETGEPLDDENIR YQIITFLIAGHETVSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH | D80I, P172G, F173W, R179L, H236I, G240R, T269V, K440C |
| | GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 56 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHGFIYSYPLAYIEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | D80I, P172G, T175Y, R179L, D182I, H236I, G240R, K440C |
| 57 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFITSYPLAYHEYMNKFQRANPDDPAYDENKR QFQEDIKVMNDLVDKIIADRKASGEQSDDLLTIMLNGKDPETGEPLDDENI RYQIITFLIAGHETVSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | D80I, R179L, D182H, H236I, T269V, K440C |
| 58 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFITSYPLAYIEYMNKFQRANPDDPAYDENKRQ FQEDIKVMNDLVDKIIADRKASGEQSDDLLTIMLNGKDPETGEPLDDENIR YQIITFLIAGHETVSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK | D80I, R179L, D182I, H236I, T269V, K440C |
| | GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 59 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFITSYPLAYDEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | D80I, R179L, H236I, K440C |
| 60 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKVSGEQSDDLLTIMLNRKDPETGEPLDDENIR YQIITFLIAGHETVSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | D80I, T175Y, A225V, H236I, G240R, T269V, K440C |
| 61 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP | D80I, T175Y, H236I, G240R, K440C |
| | STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 62 | AIKEMPQPKTFGELKNLPLLNTDKPVQALMKIADELGEIFKFEAPGRVTRYL SSQRLIKEACDESRFDKNLGQALIFLRIFEGDGLATSWTHEKNWKKAHNILL PSFSQQAMKGYHAMMVDIAVQLVQKWERLNADEHIEVPEDMTRLTLDTI GLCGFNYRFNSFYRDQPHPWIYSYPRAYDEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | K76I, D80I, F173W, T175Y, H236I, K440C |
| 63 | AIKEMPQPKTFGELKNLPLLNTDKPVQALMKIADELGEIFKFEAPGRVTRYL SSQRLIKEACDESRFDKNLGQALIFLRIFEGDGLATSWTHEKNWKKAHNILL PSFSQQAMKGYHAMMVDIAVQLVQKWERLNADEHIEVPEDMTRLTLDTI GLCGFNYRFNSFYRDQPHPFITSYPRAYDEYMVKFQRANPDDPAYDENKR QFQEDIKVMNDLVDKIIADRKVSGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | K76I, D80I, N186V, A225V, H236I, K440C |
| 64 | AIKEMPQPKTFGELKNLPLLNTDKPVQALMKIADELGEIFKFEAPGRVTRYL SSQRLIKEACDESRFDKNLGQALIFLRIFEGDGLATSWTHEKNWKKAHNILL PSFSQQAMKGYHAMMVDIAVQLVQKWERLNADEHIEVPEDMTRLTLDTI GLCGFNYRFNSFYRDQPHPFITSYPRAYDEYMVKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV | K76I, D80I, N186V, H236I, K440C |
| | LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | |
| 65 | AIKEMPQPKTFGELKNLPLLNTDKPVQALMKIADELGEIFKFEAPGRVTRYL SSQRLIKEACDESRFDKNLGQALIFLRIFEGDGLATSWTHEKNWKKAHNILL PSFSQQAMKGYHAMMVDIAVQLVQKWERLNADEHIEVPEDMTRLTLDTI GLCGFNYRFNSFYRDQPHPFITSYPLAYDEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | K76I, D80I, R179L, H236I, G240R, K440C |
| 66 | AIKEMPQPKTFGELKNLPLLNTDKPVQALMKIADELGEIFKFEAPGRVTRYL SSQRLIKEACDESRFDKNLGQALIFLRIFEGDGLATSWTHEKNWKKAHNILL PSFSQQAMKGYHAMMVDIAVQLVQKWERLNADEHIEVPEDMTRLTLDTI GLCGFNYRFNSFYRDQPHPFITSYPLAYDEYMNKFQRANPDDPAYDENKR QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | K76I, D80I, R179L, H236I, K440C |
| 67 | AIKEMPQPKTFGELKNLPLLNTDKPVQALMKIADELGEIFKFEAPGRVTRYL SSQRLIKEACDESRFDKNLGQALIFLRIFEGDGLATSWTHEKNWKKAHNILL PSFSQQAMKGYHAMMVDIAVQLVQKWERLNADEHIEVPEDMTRLTLDTI GLCGFNYRFNSFYRDQPHPFITSYPLAYDEYMVKFQRANPDDPAYDENKR QFQEDIKVMNDLVDKIIADRKASGEQSDDLLTIMLNGKDPETGEPLDDENI | K76I, D80I, R179L, N186V, H236I, K440C |
| | RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLCPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | |
| 68 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, D80I, T175Y, H236I, G240R, K440C |
| 69 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYHEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, K76I, D80I, T175Y, D182H, H236I, G240R, K440C |
| 70 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI | Q73W, K76I, D80I, T175Y, D182H, N186V, A225V, H236I, G240R, K440C |
| | GLCGFNYRFNSFYRDQPHPFIYSYPRAYHEYMVKFQRANPDDPAYDENKR QFQEDIKVMNDLVDKIIADRKVSGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | |
| 71 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYHEYMVKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, K76I, D80I, T175Y, D182H, N186V, H236I, G240R, K440C |
| 72 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYIEYMNKFQRANPDDPAYDENKRQ FQEDIKVMNDLVDKIIADRKVSGEQSDDLLTIMLNGKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, K76I, D80I, T175Y, D182I, A225V, H236I, K440C |
| 73 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYIEYMNKFQRANPDDPAYDENKRQ FQEDIKVMNDLVDKIIADRKASGEQSDDLLTIMLNGKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, K76I, D80I, T175Y, D182I, H236I, K440C |
| 74 | AIKEMPQPKTFGELKNLPLLNTDKPVQALMKIADELGEIFKFEAPGRVTRYL SSQRLIKEACDESRFDKNLGWALIFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYHEYMNKFQRANPDDPAYDENKR QFQEDIKVMNDLVDKIIADRKASGEQSDDLLTIMLNRKDPETGEPLDDENIR YQIITFLIAGHETTSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP NQPKTYVQHVMEQDGKKLIELLDQGAHFYICGDGSQMAPAVEATLMKSY ADVHQVSEADARLWLQQLEEKGRYAKDVWAG | Q73W, K76I, D80I, T175Y, R179L, D182H, H236I, G240R, K440C |
| 75 | AIKEMPQPKTFGELKNLPLLNTDKPVQALMKIADELGEIFKFEAPGRVTRYL SSQRLIKEACDESRFDKNLGLALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPLAYDEYMNKFQRANPDDPAYDENKR QFQEDIKVMNDLVDKIIADRKASGEQSDDLLTIMLNRKDPETGEPLDDENIR YQIITFLIAGHETVSGLLSFALYFLVKNPHVLQKAAEEAARVLVDPVPSYKQ VKQLKYVGMVLNEALRLWPTAPAFSLYAKEDTVLGGEYPLEKGDELMVLI PQLHRDKTIWGDDVEEFRPERFENPSAIPQHAFKPFGNGQRACIGQQFALHE ATLVLGMMLKHFDFEDHTNYELDIKETLTLCPEGFVVKAKSKKIPLGGIPSP STEQSAKKVRKKAENAHNTPLLVLYGSNMGTAEGTARDLADIAMSKGFAP QVATLDSHAGNLPREGAVLIVTASYNGHPPDNAKQFVDWLDQASADEVK GVRYSVFGCGDKNWATTYQKVPAFIDETLAAKGAENIADRGEADASDDFE GTYEEWREHMWSDVAAYFNLDIENSEDNKSTLSLQFVDSAADMPLAKMH GAFSTNVVASKELQQPGSARSTRHLEIELPKEASYQEGDHLGVIPRNYEGIV NRVTARFGLDASQQIRLEAEEEKLAHLPLAKTVSVEELLQYVELQDPVTRT QLRAMAAKTVCPPHKVELEALLEKQAYKEQVLAKRLTMLELLEKYPACE MKFSEFIALLPSIRPRYYSISSSPRVDEKQASITVSVVSGEAWSGYGEYKGIA SNYLAELQEGDTITCFISTPQSEFTLPKDPETPLIMVGPGTGVAPFRGFVQAR KQLKEQGQSLGEAHLYFGCRSPHEDYLYQEELENAQSEGIITLHTAFSRMP | Q73L, D80I, T175Y, R179L, H236I, G240R, T269V, K440C |
| | NQPKTYVQHVMEQDGKKLIELLDQGAHFYICGDGSQMAPAVEATLMKSY ADVHQVSEADARLWLQQLEEKGRYAKDVWAG | |
| 76 | TIKEMPQPKTFGELKNLPLLNTDKPVQALMKIADELGEIFKFEAPGRVTRYL SSQRLIKEACDESRFDKNLSQALKFVRDFFGDGLFTSWTHEKNWKKAHNIL LPSFSQQAMKGYHAMMVDIAVQLVQKWERLNADEHIEVPEDMTRLTLDTI GLCGFNYRFNSFYRDQPHPFITSMVRALDEAMNKLQRANPDDPAYDENKR QFQEDIKVMNDLVDKIIADRKASGEQSDDLLTHMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLKPEGFVVKAKSKKIPLGGI | n.a. |
| 77 | AIKEMPQPKTFGELKNLPLLNTDKPVQALMKIADELGEIFKFEAPGRVTRYL SSQRLIKEACDESRFDKNLSQALKFVRDFAGDGLFTSWTHEKNWKKAHNI LLPSFSQQAMKGYHAMMVDIAVQLVQKWERLNADEHIEVPEDMTRLTLD TIGLCGFNYRFNSFYRDQPHPFITSMVRALDEAMNKLQRANPDDPAYDEN KRQFQEDIKVMNDLVDKIIADRKASGEQSDDLLTHMLNGKDPETGEPLDD ENIRYQIITFLIAGHETTSGLLSFALYFLVKNPHVLQKAAEEAARVLVDPVPS YKQVKQLKYVGMVLNEALRLWPTAPAFSLYAKEDTVLGGEYPLEKGDEL MVLIPQLHRDKTIWGDDVEEFRPERFENPSAIPQHAFKPFGNGQRACIGQQF ALHEATLVLGMMLKHFDFEDHTNYELDIKETLTLKPEGFVVKAKSKKIPLG GIPSPSTEQSAKKVRKKAENAHNTPLLVLYGSNMGTAEGTARDLADIAMS KGFAPQVATLDSHAGNLPREGAVLIVTASYNGHPPDNAKQFVDWLDQASA DEVKGVRYSVFGCGDKNWATTYQKVPAFIDETLAAKGAENIADRGEADA SDDFEGTYEEWREHMWSDVAAYFNLDIENSEDNKSTLSLQFVDSAADMPL AKMHGAFSTNVVASKELQQPGSARSTRHLEIELPKEASYQEGDHLGVIPRN YEGIVNRVTARFGLDASQQIRLEAEEEKLAHLPLAKTVSVEELLQYVELQD PVTRTQLRAMAAKTVCPPHKVELEALLEKQAYKEQVLAKRLTMLELLEKY PACEMKFSEFIALLPSIRPRYYSISSSPRVDEKQASITVSVVSGEAWSGYGEY KGIASNYLAELQEGDTITCFISTPQSEFTLPKDPETPLIMVGPGTGVAPFRGF VQARKQLKEQGQSLGEAHLYFGCRSPHEDYLYQEELENAQSEGIITLHTAF SRMPNQPKTYVQHVMEQDGKKLIELLDQGAHFYICGDGSQMAPAVEATL MKSYADVHQVSEADARLWLQQLEEKGRYAKDVWAG | n.a. |
| 78 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFITSYPRAYHEYMNKFQRANPDDPAYDENKR QFQEDIKVMNDLVDKIIADRKVSGEQSDDLLTHMLNVKDPETGEPLDDENI RYQIITFLIAGHETVSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLKPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | D80I, D182H, A225V, G240V, T269V |
| 79 | AIKEMPQPKTFGELKNLPLLNTDKPVQALMKIADELGEIFKFEAPGRVTRYL SSQRLIKEACDESRFDKNLGWALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFITSYPRAYDEYMVKFQRANPDDPAYDENKR QFQEDIKVMNDLVDKIIADRKASGEQSDDLLTHMLNVKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL | Q73W, D80I, N186V, G240V |
| | HEATLVLGMMLKHFDFEDHTNYELDIKETLTLKPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | |
| 80 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFITSYPRAYDEYMNKFQRANPDDPAYDENKR QFQEDIKVMNDLVDKIIADRKASGEQSDDLLTHMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLKPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | D80I |
| 81 | AIKEMPQPKTFGELKNLPLLNTDKPVQALMKIADELGEIFKFEAPGRVTRYL SSQRLIKEACDESRFDKNLGQALKFLRDFEGDGLATSWTHEKNWKKAHNI LLPSFSQQAMKGYHAMMVDIAVQLVQKWERLNADEHIEVPEDMTRLTLD TIGLCGFNYRFNSFYRDQPHPFITSYPRAYDEYMNKFQRANPDDPAYDENK RQFQEDIKVMNDLVDKIIADRKASGEQSDDLLTHMLNGKDPETGEPLDDE NIRYQIITFLIAGHETTSGLLSFALYFLVKNPHVLQKAAEEAARVLVDPVPS YKQVKQLKYVGMVLNEALRLWPTAPAFSLYAKEDTVLGGEYPLEKGDEL MVLIPQLHRDKTIWGDDVEEFRPERFENPSAIPQHAFKPFGNGQRACIGQQF ALHEATLVLGMMLKHFDFEDHTNYELDIKETLTLCPEGFVVKAKSKKIPLG GIPSPSTEQSAKKVRKKAENAHNTPLLVLYGSNMGTAEGTARDLADIAMS KGFAPQVATLDSHAGNLPREGAVLIVTASYNGHPPDNAKQFVDWLDQASA DEVKGVRYSVFGCGDKNWATTYQKVPAFIDETLAAKGAENIADRGEADA SDDFEGTYEEWREHMWSDVAAYFNLDIENSEDNKSTLSLQFVDSAADMPL AKMHGAFSTNVVASKELQQPGSARSTRHLEIELPKEASYQEGDHLGVIPRN YEGIVNRVTARFGLDASQQIRLEAEEEKLAHLPLAKTVSVEELLQYVELQD PVTRTQLRAMAAKTVCPPHKVELEALLEKQAYKEQVLAKRLTMLELLEKY PACEMKFSEFIALLPSIRPRYYSISSSPRVDEKQASITVSVVSGEAWSGYGEY KGIASNYLAELQEGDTITCFISTPQSEFTLPKDPETPLIMVGPGTGVAPFRGF VQARKQLKEQGQSLGEAHLYFGCRSPHEDYLYQEELENAQSEGIITLHTAF SRMPNQPKTYVQHVMEQDGKKLIELLDQGAHFYICGDGSQMAPAVEATL MKSYADVHQVSEADARLWLQQLEEKGRYAKDVWAG | K440C |
| 82 | AIKEMPQPKTFGELKNLPLLNTDKPVQALMKIADELGEIFKFEAPGRVTRYL SSQRLIKEACDESRFDKNLGQALKFLRDFEGDGLATSWTHEKNWKKAHNI LLPSFSQQAMKGYHAMMVDIAVQLVQKWERLNADEHIEVPEDMFRLTLD TIGLCGFNYRFNSFYRDQPHPFITSYWRAYDEYMNKLQRANPDDPAYDEN KRQFQEDIKVMNDLVDKIIADRKASGEQSDDLLTHMLNGKDPETGEPLDD ENIRYQIITFLIAGHETTSGLLSFALYFLVKNPHVLQKAAEEAARVLVDPVPS | T146F, P178W, F188L, G457S, I458G, P459-N476:VKMVR VQAIEKNRYR A--, A477N |
| | YKQVKQLKYVGMVLNEALRLWPTAPAFSLYAKEDTVLGGEYPLEKGDEL MVLIPQLHRDKTIWGDDVEEFRPERFENPSAIPQHAFKPFGNGQRACIGQQF ALHEATLVLGMMLKHFDFEDHTNYELDIKETLTLKPEGFVVKAKSKKIPLG SGVKMVRVQAIEKNRYRANHNTPLLVLYGSNMGTAEGTARDLADIAMSK GFAPQVATLDSHAGNLPREGAVLIVTASYNGHPPDNAKQFVDWLDQASAD EVKGVRYSVFGCGDKNWATTYQKVPAFIDETLAAKGAENIADRGEADAS DDFEGTYEEWREHMWSDVAAYFNLDIENSEDNKSTLSLQFVDSAADMPLA KMHGAFSTNVVASKELQQPGSARSTRHLEIELPKEASYQEGDHLGVIPRNY EGIVNRVTARFGLDASQQIRLEAEEEKLAHLPLAKTVSVEELLQYVELQDP VTRTQLRAMAAKTVCPPHKVELEALLEKQAYKEQVLAKRLTMLELLEKYP ACEMKFSEFIALLPSIRPRYYSISSSPRVDEKQASITVSVVSGEAWSGYGEYK GIASNYLAELQEGDTITCFISTPQSEFTLPKDPETPLIMVGPGTGVAPFRGFV QARKQLKEQGQSLGEAHLYFGCRSPHEDYLYQEELENAQSEGIITLHTAFS RMPNQPKTYVQHVMEQDGKKLIELLDQGAHFYICGDGSQMAPAVEATLM KSYADVHQVSEADARLWLQQLEEKGRYAKDVWAG | |
| 83 | AIKEMPQPKTFGELKNLPLLNTDKPVQALMKIADELGEIFKFEAPGRVTRYL SSQRLIKEACDESRFDKNLGQALKFLRDFEGDGLATSWTHEKNWKKAHNI LLPSFSQQAMKGYHAMMVDIAVQLVQKWERLNADEHIEVPEDMFRLTLD TIGLCGFNYRFNSFYRDQPHPFITSYWRAYDEYMNKLQRANPDDPAYDEN KRQFQEDIKVMNDLVDKIIADRKASGEQSDDLLTHMLNGKDPETGEPLDD ENIRYQIITFLIAGHETTSGLLSFALYFLVKNPHVLQKAAEEAARVLVDPVPS YKQVKQLKYVGMVLNEALRLWPTAPAFSLYAKEDTVLGGEYPLEKGDEL MVLIPQLHRDKTIWGDDVEEFRPERFENPSAIPQHAFKPFGNGQRACIGQQF ALHEATLVLGMMLKHFDFEDHTNYELDIKETLTLKPEGFVVKAKSKKIPLG SGPSPSTEQSAKKVRKKAENNHNTPLLVLYGSNMGTAEGTARDLADIAMS KGFAPQVATLDSHAGNLPREGAVLIVTASYNGHPPDNAKQFVDWLDQASA DEVKGVRYSVFGCGDKNWATTYQKVPAFIDETLAAKGAENIADRGEADA SDDFEGTYEEWREHMWSDVAAYFNLDIENSEDNKSTLSLQFVDSAADMPL AKMHGAFSTNVVASKELQQPGSARSTRHLEIELPKEASYQEGDHLGVIPRN YEGIVNRVTARFGLDASQQIRLEAEEEKLAHLPLAKTVSVEELLQYVELQD PVTRTQLRAMAAKTVCPPHKVELEALLEKQAYKEQVLAKRLTMLELLEKY PACEMKFSEFIALLPSIRPRYYSISSSPRVDEKQASITVSVVSGEAWSGYGEY KGIASNYLAELQEGDTITCFISTPQSEFTLPKDPETPLIMVGPGTGVAPFRGF VQARKQLKEQGQSLGEAHLYFGCRSPHEDYLYQEELENAQSEGIITLHTAF SRMPNQPKTYVQHVMEQDGKKLIELLDQGAHFYICGDGSQMAPAVEATL MKSYADVHQVSEADARLWLQQLEEKGRYAKDVWAG | T146F, P178W, F188L, G457S, I458G, A477N |
| 84 | AIKEMPQPKTFGELKNLPLLNTDKPVQALMKIADELGEIFKFEAPGRVTRYL SSQRLIKEACDESRFDKNLGQALKFLRDFEGDGLATSWTHEKNWKKAHNI LLPSFSQQAMKGYHAMMVDIAVQLVQKWERLNADEHIEVPEDMFRLTLD TIGLCGFNYRFNSFYRDQPHPFITSYWRAYDEYMNKLQRANPDDPAYDEN KRQFQEDIKVMNDLVDKIIADRKASGEQSDDLLTHMLNGKDPETGEPLDD ENIRYQIITFLIAGHETTSGLLSFALYFLVKNPHVLQKAAEEAARVLVDPVPS YKQVKQLKYVGMVLNEALRLWPTAPAFSLYAKEDTVLGGEYPLEKGDEL MVLIPQLHRDKTIWGDDVEEFRPERFENPSAIPQHAFKPFGNGQRACIGQQF ALHEATLVLGMMLKHFDFEDHTNYELDIKETLTLKPEGFVVKAKSKKIPLG SGTARKGKGAFCNGQKLQVNHNTPLLVLYGSNMGTAEGTARDLADIAMS KGFAPQVATLDSHAGNLPREGAVLIVTASYNGHPPDNAKQFVDWLDQASA DEVKGVRYSVFGCGDKNWATTYQKVPAFIDETLAAKGAENIADRGEADA SDDFEGTYEEWREHMWSDVAAYFNLDIENSEDNKSTLSLQFVDSAADMPL AKMHGAFSTNVVASKELQQPGSARSTRHLEIELPKEASYQEGDHLGVIPRN YEGIVNRVTARFGLDASQQIRLEAEEEKLAHLPLAKTVSVEELLQYVELQD PVTRTQLRAMAAKTVCPPHKVELEALLEKQAYKEQVLAKRLTMLELLEKY PACEMKFSEFIALLPSIRPRYYSISSSPRVDEKQASITVSVVSGEAWSGYGEY KGIASNYLAELQEGDTITCFISTPQSEFTLPKDPETPLIMVGPGTGVAPFRGF VQARKQLKEQGQSLGEAHLYFGCRSPHEDYLYQEELENAQSEGIITLHTAF SRMPNQPKTYVQHVMEQDGKKLIELLDQGAHFYICGDGSQMAPAVEATL MKSYADVHQVSEADARLWLQQLEEKGRYAKDVWAG | T146F, P178W, F188L, G457S, I458G, P459-N476:TARKGK GAFCNGQKL QV-, A477N |
| 85 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR | D80I, T175Y, H236I, K440T |
| | QFQEDIKVMNDLVDKIIADRKASGEQSDDLLTIMLNGKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLTPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | |
| 86 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFIYSYPRAYDEYMNKFQRANPDDPAYDENKR QFQEDIKVMNDLVDKIIADRKASGEQSDDLLTKMLNVKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLKPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | D80I, T175Y, H236K, G240V |
| 87 | AIKEMPQPKTFGELKNLPLLNTDKPVQALMKIADELGEIFKFEAPGRVTRYL SSQRLIKEACDESRFDKNLGQALKFLRDFEGDGLATSWTHEKNWKKAHNI LLPSFSQQAMKGYHAMMVDIAVQLVQKWERLNADEHIEVPEDMTRLTLD TIGLCGFNYRFNSFYRDQPHPFITSYPRAYDEYMNKFQRANPDDPAYDENK RQFQEDIKVMNDLVDKIIADRKASGEQSDDLLTHMLNAKDPETGEPLDDE NIRYQIITFLIAGHETTSGLLSFALYFLVKNPHVLQKAAEEAARVLVDPVPS YKQVKQLKYVGMVLNEALRLWPTAPAFSLYAKEDTVLGGEYPLEKGDEL MVLIPQLHRDKTIWGDDVEEFRPERFENPSAIPQHAFKPFGNGQRACIGQQF ALHEATLVLGMMLKHFDFEDHTNYELDIKETLTLCPEGFVVKAKSKKIPLG GIPSPSTEQSAKKVRKKAENAHNTPLLVLYGSNMGTAEGTARDLADIAMS KGFAPQVATLDSHAGNLPREGAVLIVTASYNGHPPDNAKQFVDWLDQASA DEVKGVRYSVFGCGDKNWATTYQKVPAFIDETLAAKGAENIADRGEADA SDDFEGTYEEWREHMWSDVAAYFNLDIENSEDNKSTLSLQFVDSAADMPL AKMHGAFSTNVVASKELQQPGSARSTRHLEIELPKEASYQEGDHLGVIPRN YEGIVNRVTARFGLDASQQIRLEAEEEKLAHLPLAKTVSVEELLQYVELQD PVTRTQLRAMAAKTVCPPHKVELEALLEKQAYKEQVLAKRLTMLELLEKY PACEMKFSEFIALLPSIRPRYYSISSSPRVDEKQASITVSVVSGEAWSGYGEY KGIASNYLAELQEGDTITCFISTPQSEFTLPKDPETPLIMVGPGTGVAPFRGF VQARKQLKEQGQSLGEAHLYFGCRSPHEDYLYQEELENAQSEGIITLHTAF SRMPNQPKTYVQHVMEQDGKKLIELLDQGAHFYICGDGSQMAPAVEATL MKSYADVHQVSEADARLWLQQLEEKGRYAKDVWAG | G240A, K440C |
| 88 | AIKEMPQPKTFGELKNLPLLNTDKPVQALMKIADELGEIFKFEAPGRVTRYL SSQRLIKEACDESRFDKNLGLALKFLRDFEGDGLATSWTHEKNWKKAHNI | Q73L, G240R, K440T |
| | LLPSFSQQAMKGYHAMMVDIAVQLVQKWERLNADEHIEVPEDMTRLTLD TIGLCGFNYRFNSFYRDQPHPFITSYPRAYDEYMNKFQRANPDDPAYDENK RQFQEDIKVMNDLVDKIIADRKASGEQSDDLLTHMLNRKDPETGEPLDDE NIRYQIITFLIAGHETTSGLLSFALYFLVKNPHVLQKAAEEAARVLVDPVPS YKQVKQLKYVGMVLNEALRLWPTAPAFSLYAKEDTVLGGEYPLEKGDEL MVLIPQLHRDKTIWGDDVEEFRPERFENPSAIPQHAFKPFGNGQRACIGQQF ALHEATLVLGMMLKHFDFEDHTNYELDIKETLTLTPEGFVVKAKSKKIPLG GIPSPSTEQSAKKVRKKAENAHNTPLLVLYGSNMGTAEGTARDLADIAMS KGFAPQVATLDSHAGNLPREGAVLIVTASYNGHPPDNAKQFVDWLDQASA DEVKGVRYSVFGCGDKNWATTYQKVPAFIDETLAAKGAENIADRGEADA SDDFEGTYEEWREHMWSDVAAYFNLDIENSEDNKSTLSLQFVDSAADMPL AKMHGAFSTNVVASKELQQPGSARSTRHLEIELPKEASYQEGDHLGVIPRN YEGIVNRVTARFGLDASQQIRLEAEEEKLAHLPLAKTVSVEELLQYVELQD PVTRTQLRAMAAKTVCPPHKVELEALLEKQAYKEQVLAKRLTMLELLEKY PACEMKFSEFIALLPSIRPRYYSISSSPRVDEKQASITVSVVSGEAWSGYGEY KGIASNYLAELQEGDTITCFISTPQSEFTLPKDPETPLIMVGPGTGVAPFRGF VQARKQLKEQGQSLGEAHLYFGCRSPHEDYLYQEELENAQSEGIITLHTAF SRMPNQPKTYVQHVMEQDGKKLIELLDQGAHFYICGDGSQMAPAVEATL MKSYADVHQVSEADARLWLQQLEEKGRYAKDVWAG | |
| 89 | AIKEMPQPKTFGELKNLPLLNTDKPVQALMKIADELGEIFKFEAPGRVTRYL SSQRLIKEACDESRFDKNLGLALKFLRDFEGDGLATSWTHEKNWKKAHNI LLPSFSQQAMKGYHAMMVDIAVQLVQKWERLNADEHIEVPEDMTRLTLD TIGLCGFNYRFNSFYRDQPHPFITSYPRAYDEYMNKFQRANPDDPAYDENK RQFQEDIKVMNDLVDKIIADRKASGEQSDDLLTTMLNVKDPETGEPLDDEN IRYQIITFLIAGHETTSGLLSFALYFLVKNPHVLQKAAEEAARVLVDPVPSY KQVKQLKYVGMVLNEALRLWPTAPAFSLYAKEDTVLGGEYPLEKGDELM VLIPQLHRDKTIWGDDVEEFRPERFENPSAIPQHAFKPFGNGQRACIGQQFA LHEATLVLGMMLKHFDFEDHTNYELDIKETLTLCPEGFVVKAKSKKIPLGG IPSPSTEQSAKKVRKKAENAHNTPLLVLYGSNMGTAEGTARDLADIAMSK GFAPQVATLDSHAGNLPREGAVLIVTASYNGHPPDNAKQFVDWLDQASAD EVKGVRYSVFGCGDKNWATTYQKVPAFIDETLAAKGAENIADRGEADAS DDFEGTYEEWREHMWSDVAAYFNLDIENSEDNKSTLSLQFVDSAADMPLA KMHGAFSTNVVASKELQQPGSARSTRHLEIELPKEASYQEGDHLGVIPRNY EGIVNRVTARFGLDASQQIRLEAEEEKLAHLPLAKTVSVEELLQYVELQDP VTRTQLRAMAAKTVCPPHKVELEALLEKQAYKEQVLAKRLTMLELLEKYP ACEMKFSEFIALLPSIRPRYYSISSSPRVDEKQASITVSVVSGEAWSGYGEYK GIASNYLAELQEGDTITCFISTPQSEFTLPKDPETPLIMVGPGTGVAPFRGFV QARKQLKEQGQSLGEAHLYFGCRSPHEDYLYQEELENAQSEGIITLHTAFS RMPNQPKTYVQHVMEQDGKKLIELLDQGAHFYICGDGSQMAPAVEATLM KSYADVHQVSEADARLWLQQLEEKGRYAKDVWAG | Q73L, H236T, G240V, K440C |
| 90 | AIKEMPQPKTFGELKNLPLLNTDKPVQALMKIADELGEIFKFEAPGRVTRYL SSQRLIKEACDESRFDKNLGWALKFLRDFEGDGLATSWTHEKNWKKAHNI LLPSFSQQAMKGYHAMMVDIAVQLVQKWERLNADEHIEVPEDMTRLTLD TIGLCGFNYRFNSFYRDQPHPFIRSYPRAYDEYMNKFQRANPDDPAYDENK RQFQEDIKVMNDLVDKIIADRKASGEQSDDLLTHMLNVKDPETGEPLDDE NIRYQIITFLIAGHETTSGLLSFALYFLVKNPHVLQKAAEEAARVLVDPVPS YKQVKQLKYVGMVLNEALRLWPTAPAFSLYAKEDTVLGGEYPLEKGDEL MVLIPQLHRDKTIWGDDVEEFRPERFENPSAIPQHAFKPFGNGQRACIGQQF ALHEATLVLGMMLKHFDFEDHTNYELDIKETLTLKPEGFVVKAKSKKIPLG GIPSPSTEQSAKKVRKKAENAHNTPLLVLYGSNMGTAEGTARDLADIAMS KGFAPQVATLDSHAGNLPREGAVLIVTASYNGHPPDNAKQFVDWLDQASA DEVKGVRYSVFGCGDKNWATTYQKVPAFIDETLAAKGAENIADRGEADA SDDFEGTYEEWREHMWSDVAAYFNLDIENSEDNKSTLSLQFVDSAADMPL AKMHGAFSTNVVASKELQQPGSARSTRHLEIELPKEASYQEGDHLGVIPRN YEGIVNRVTARFGLDASQQIRLEAEEEKLAHLPLAKTVSVEELLQYVELQD PVTRTQLRAMAAKTVCPPHKVELEALLEKQAYKEQVLAKRLTMLELLEKY PACEMKFSEFIALLPSIRPRYYSISSSPRVDEKQASITVSVVSGEAWSGYGEY KGIASNYLAELQEGDTITCFISTPQSEFTLPKDPETPLIMVGPGTGVAPFRGF VQARKQLKEQGQSLGEAHLYFGCRSPHEDYLYQEELENAQSEGIITLHTAF SRMPNQPKTYVQHVMEQDGKKLIELLDQGAHFYICGDGSQMAPAVEATL MKSYADVHQVSEADARLWLQQLEEKGRYAKDVWAG | Q73W, T175R, G240V |
| 91 | AIKEMPQPKTFGELKNLPLLNTDKPVQALMKIADELGEIFKFEAPGRVTRYL SSQRLIKEACDESRFDKNLGQALKFLRDFEGDGLATSWTHEKNWKKAHNI LLPSFSQQAMKGYHAMMVDIAVQLVQKWERLNADEHIEVPEDMTRLTLD TIGLCGFNYRFNSFYRDQPHPFIYSYPRAYDEYMNKFQRANPDDPAYDENK RQFQEDIKVMNDLVDKIIADRKASGEQSDDLLTKMLNAKDPETGEPLDDE NIRYQIITFLIAGHETTSGLLSFALYFLVKNPHVLQKAAEEAARVLVDPVPS YKQVKQLKYVGMVLNEALRLWPTAPAFSLYAKEDTVLGGEYPLEKGDEL MVLIPQLHRDKTIWGDDVEEFRPERFENPSAIPQHAFKPFGNGQRACIGQQF ALHEATLVLGMMLKHFDFEDHTNYELDIKETLTLKPEGFVVKAKSKKIPLG GIPSPSTEQSAKKVRKKAENAHNTPLLVLYGSNMGTAEGTARDLADIAMS KGFAPQVATLDSHAGNLPREGAVLIVTASYNGHPPDNAKQFVDWLDQASA DEVKGVRYSVFGCGDKNWATTYQKVPAFIDETLAAKGAENIADRGEADA SDDFEGTYEEWREHMWSDVAAYFNLDIENSEDNKSTLSLQFVDSAADMPL AKMHGAFSTNVVASKELQQPGSARSTRHLEIELPKEASYQEGDHLGVIPRN YEGIVNRVTARFGLDASQQIRLEAEEEKLAHLPLAKTVSVEELLQYVELQD PVTRTQLRAMAAKTVCPPHKVELEALLEKQAYKEQVLAKRLTMLELLEKY PACEMKFSEFIALLPSIRPRYYSISSSPRVDEKQASITVSVVSGEAWSGYGEY KGIASNYLAELQEGDTITCFISTPQSEFTLPKDPETPLIMVGPGTGVAPFRGF VQARKQLKEQGQSLGEAHLYFGCRSPHEDYLYQEELENAQSEGIITLHTAF SRMPNQPKTYVQHVMEQDGKKLIELLDQGAHFYICGDGSQMAPAVEATL MKSYADVHQVSEADARLWLQQLEEKGRYAKDVWAG | T175Y, H236K, G240A |
| 92 | AIKEMPQPKTFGELKNLPLLNTDKPVQALMKIADELGEIFKFEAPGRVTRYL SSQRLIKEACDESRFDKNLGQALKFLRIFEGDGLATSWTHEKNWKKAHNIL LPSFSQQAMKGYHAMMVDIAVQLVQKWERLNADEHIEVPEDMTRLTLDTI GLCGFNYRFNSFYRDQPHPFITSYPRAYDEYMNKFQRANPDDPAYDENKR QFQEDIKVMNDLVDKIIADRKASGEQSDDLLTTMLNAKDPETGEPLDDENI RYQIITFLIAGHETTSGLLSFALYFLVKNPHVLQKAAEEAARVLVDPVPSYK QVKQLKYVGMVLNEALRLWPTAPAFSLYAKEDTVLGGEYPLEKGDELMV LIPQLHRDKTIWGDDVEEFRPERFENPSAIPQHAFKPFGNGQRACIGQQFAL HEATLVLGMMLKHFDFEDHTNYELDIKETLTLKPEGFVVKAKSKKIPLGGI PSPSTEQSAKKVRKKAENAHNTPLLVLYGSNMGTAEGTARDLADIAMSKG FAPQVATLDSHAGNLPREGAVLIVTASYNGHPPDNAKQFVDWLDQASADE VKGVRYSVFGCGDKNWATTYQKVPAFIDETLAAKGAENIADRGEADASD DFEGTYEEWREHMWSDVAAYFNLDIENSEDNKSTLSLQFVDSAADMPLAK MHGAFSTNVVASKELQQPGSARSTRHLEIELPKEASYQEGDHLGVIPRNYE GIVNRVTARFGLDASQQIRLEAEEEKLAHLPLAKTVSVEELLQYVELQDPV TRTQLRAMAAKTVCPPHKVELEALLEKQAYKEQVLAKRLTMLELLEKYPA CEMKFSEFIALLPSIRPRYYSISSSPRVDEKQASITVSVVSGEAWSGYGEYKG IASNYLAELQEGDTITCFISTPQSEFTLPKDPETPLIMVGPGTGVAPFRGFVQ ARKQLKEQGQSLGEAHLYFGCRSPHEDYLYQEELENAQSEGIITLHTAFSR MPNQPKTYVQHVMEQDGKKLIELLDQGAHFYICGDGSQMAPAVEATLMK SYADVHQVSEADARLWLQQLEEKGRYAKDVWAG | D80I, H236T, G240A |
| 93 | MKHHHHHHPMSDYDIPTTENLYFEGAM | n.a. |

### DEFINITIONS

Unless otherwise defined, all scientific and technical terms used in the description, figures and claims have their ordinary meaning as commonly understood by one of ordinary skill in the art. All publications, patent applications, patents, and other references mentioned herein are incorporated by reference in their entirety. In case of conflict, the present specification, including definitions, will prevail. If two or more documents incorporated by reference include conflicting and/or inconsistent disclosure with respect to each other, then the document having the later effective date shall control. The materials, methods, and examples are illustrative only and not intended to be limiting. Unless stated otherwise, the following terms used in this document, including the description and claims, have the definitions given below. The terms "comprising", "including", "containing", "having" etc. shall be read expansively or openended and without limitation. Singular forms such as "a", "an" or "the" include plural references unless the context clearly indicates otherwise. Unless otherwise indicated, the term "at least" preceding a series of elements is to be understood to refer to every element in the series. The terms "at least one" and "at least one of" include for example, one, two, three, four, five, six, seven, eight, nine, ten or more elements.

It is furthermore understood that slight variations above and below a stated range can be used to achieve substantially the same results as a value within the range. Also, unless indicated otherwise, the disclosure of ranges is intended as a continuous range including every value between the minimum and maximum values.

Where protein or amino acid sequences are provided throughout the application it is also understood by the skilled person that single or multiple amino acids may be exchanged by amino acids with similar properties to achieve substantially the same effect, i.e.an equivalent result. The skilled person furthermore knows that a defined protein or amino acid sequence may be encoded by various nucleic acid sequences. For a given amino acid sequence as defined herein, each of the countable nucleic acid sequences encoding the specific amino acid sequence shall be deemed to be disclosed herein. Where nucleic acid sequences are provided throughout the application it is furthermore understood that silent mutations may be introduced.

The terms **"peptide", "polypeptide",** and **"protein"** are used interchangeably herein, and refer to a compound which comprises at least two amino acid residues covalently linked by at least one peptide bond. No limitation is placed on the maximum number of amino acids that can comprise a peptide's sequence. A "peptide" may comprise without limitation modified amino acids, non naturally-occuring amino acids and/or D amino acids. Unless otherwise indicated, a particular peptide sequence also encompasses variants wherein at least one amino acid has been replaced by an amino acid which is characterized by similar structural properties. A "peptide" may be a natural peptide, a recombinant peptide, a synthetic peptide, or a combination thereof. A "peptide" may be, for example, a biologically active fragment, an oligopeptide, a homodimer, a heterodimer, a peptide variant, a modified peptide, a peptide derivative, a peptide analog, a fusion protein, among others.

The term **"amino acid"** or **"amino acid residue" ("aa")** as used herein typically refers to a naturally-occuring amino acid but may also refer to a non naturally-occuring amino acid. The term typically refers to an L-amino acid but may also encompass a D-amino acid. An amino acid may or may not be modified as described elsewhere herein. The one letter code is used herein to refer to the respective amino acid. As used herein, a **"charged amino acid"** is an amino acid which is negatively charged or positively charged. **"Negatively charged amino acids"** are aspartic acid (D) and glutamic acid (E). **"Positively charged amino acids"** are arginine (R) lysine (K) and histidine (H). **"Polar amino acids"** are all amino acids that form hydrogen bonds as donors or acceptors. These are all charged amino acids and asparagine (N), glutamine (Q), serine (S), threonine (T), tyrosine (Y) and cysteine (C). **"Polar uncharged amino acids"** are asparagine (N), glutamine (Q), serine (S), threonine (T), tyrosine (Y) and cysteine (C). **"Amphiphatic amino acids"** are tryptophan (W), tyrosine (Y) and methionine (M). **"Aromatic amino acids"** are phenylalanine (F), tyrosine (Y), and tryptophan (W). **"Hydrophobic amino acids"** are glycine (G), alanine (A), valine (V), leucine (L), isoleucine (I), proline (P), phenylalanine (F), methionine (M) and cysteine. **"Small amino acids"** are glycine (G), alanine (A), serine (S), proline (P), threonine (T), aspartic acid (D) and asparagine (N).

Two amino acids are **"characterized by similar structural properties"** if (a) both are charged amino acids, preferably both are negatively charged amino or both are positively charged amino acids, (b) both are polar amino acids, (c) both are polar uncharged amino acids, (d) both are amphiphatic amino acids, (e) both are aromatic amino acids, (f) both are hydrophobic amino acids, or (g) both are small amino acids. As understood by the skilled person, where an amino acid sequence is provided throughout the disclosure, amino acids characterized by similar structural properties may be exchanged with each other to achieve substantially the same or an equivalent result.

The terms **"cytochrome P450 BM3 monooxygenase", "cytochrome P450-BM3", "P450-BM3"** (also **BM3, BM-3**) refer to the cytochrome P450 enzyme obtained from *Bacillus megaterium* that catalyzes the hydroxylation of long-chain fatty acids, alcohols, and amides, as well as the epoxidation of unsaturated fatty acids. Without being bound by theory, P450-BM3 is naturally expressed by *Bacillus megaterium strains ATCC 14581, DSM 32, JCM 2506, NBRC 15308, NCIMB 9376, NCTC 10342, VKM B-512.* Alternative names comprise *"Bifunctional cytochrome P450*/*NADPH--P450 reductase".* The P450-BM3 protein is encoded by the gene cyp102A1. Sequence(s) are accessible via UniProt Identifier P14779 (CPXB_BACMB). Different isoforms and variants may exist for the different strains and are all comprised by the term. Where a specific mutation can be exchanged without changing the described catalytic properties of the initial sequence, it is clear that the sequence having such a functionally silent mutation is equivalent with regard to the initial sequence. In addition, the protein may furthermore be subject to various modifications, e.g, synthetic or naturally occurring modifications.

The gene encoding the cytochrome P450-monooxygenases (P450-BM3) from *Bacillus megaterium* was originally amplified from genomic DNA by PCR and subsequently cloned using restriction endonucleases *NcoI* and *SacI* into the polylinker sequence of plasmid pETM11, resulting in pETM11-BM3 (Kille 2010). This procedure adds a hexa-histidine tag and an 18 amino acid linker sequence to the N-terminus of the resulting protein, which is however not essential for function. Introducing the *NcoI* site at the 5' end of the gene sequence resulted in alteration of the second codon of the P450-BM3 gene and to an amino acid exchange in the resulting protein (T1A).

An **amino acid position** is typically given by specifying the number of the respective position behind the amino acid of the wildtype using the one letter code (e.g. V78 where the wildtype has a valine at position 78). A list of comma separated letters in squared brackets specifies the **mutation or amino acid exchange.** For example, where at position 78 of the wildtype variant the valine is replaced by any one of isoleucine, leucine, or methionine, or is not mutated at all, this is abbreviated as V78[I, L, M, V]. Throughout this application **counting of amino acid positions** of the P450-BM3 amino acid sequence starts with the first amino acid that appears in the protein sequence underlying the crystal structure of the heme domain of a P450-BM3 variant (PDB ID: 2UWH; SEQ ID NO 76) that was used for designing protein libraries. This is also common practice in P450-BM3-related literature, see e.g. (Kille 2010), (Kille Sabrina 2011), (Acevedo-Rocha 2018). The N-terminal methionine is ignored such that counting starts with threonine 1, which was converted to an alanine in the cloning process of pETM11-BM3 and which is codon 1 in SEQ ID NO1-75 and SEQ ID NO 77-92 according to the SEQ IDs listed herein - in contrast to the sequences given in WO2021156200A1.

The term **"P450-BM3 variant"** refers to a **P450-BM3** comprising at least one mutation relative to SEQ ID NO 1. Where mutations of a variant are specified, these mutations are restrictive, for example a BM3 SEQ ID NO 1, D80I, H236I, K440C variant having up to six mutations is required to have the mutations D80I, H236I, K440C, and three further mutations.

A **"daughter variant"** of a **"parent variant"** is a variant having the sequence of its parent variant and at least one further mutation. A daughter variant can have multiple parent variants.

According to the IUPAC definition **"steroids"** are naturally occurring compounds and synthetic analogues, which are based on the cyclopenta[a]phenanthrene carbon skeleton, and can be partially or completely hydrogenated. Usually but not necessarily, there are methyl groups at C-10 and C-13, and often but not necessarily there is an alkyl group at C-17. By extension, one or more bond scissions, ring expansions and/or ring contractions of the skeleton may or may not have occurred. Throughout this application, steroids are numbered and rings are lettered as in the following formula:

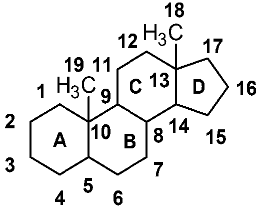

When the rings of a steroid are denoted as projections onto the plane of the paper, the formula is normally to be oriented as in the following formula:

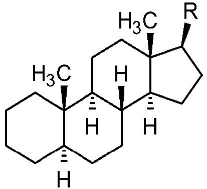

An atom or group attached to a ring depicted as in this orientation is termed **"alpha"** if it lies below the plane of the paper or **"beta"** if it lies above the plane of the paper. Examples for steroids include androgens, estrogens, and progestogens, corticosteroids, glucocorticoids, mineralocorticoids, cholesterol, estradiol, testosterone, dexamethasone, lanosterol, progesterone, medrogestone, β-sitosterol.

The term **steroid derivative** refers to molecules derived from a steroid, i.e. molecules comprising a cyclopenta[a]phenanthrene carbon skeleton.

The term **"C19 hydroxylation"** refers to the introduction of a hydroxyl group (-OH) into a steroid or steroid derivative at the position C19.

Without being bound by theory, for steroids with a **1,4-dien-3-one-A-ring** such as (17beta)-17-hydroxyandrosta-1,4-dien-3-one (synonyms e.g.: 1-dehydrotestosterone, Δ1-testosterone) or androsta-1,4-dien-3,17-dione (synonym: ADD) a C19-hydroxylation is believed to lead to an instable intermediate undergoing immediate aromatization and formation of estradiol or estrone and their derivatives.

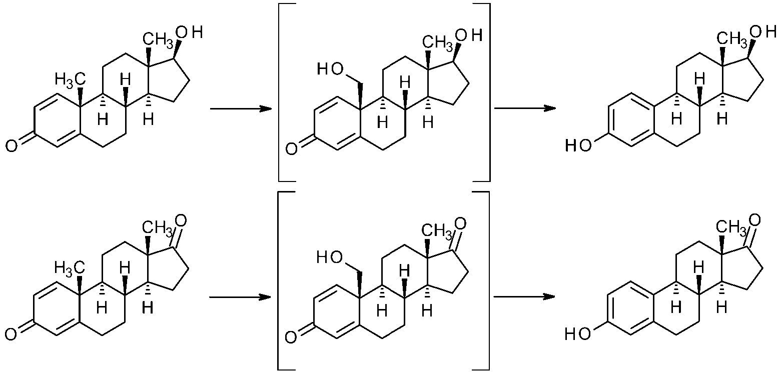

Hence for steroids with a 1,4-dien-3-one-A-ring the wording cytochrome P450 BM3 monooxygenase (BM3) variant for catalyzing the C19 hydroxylation of a steroid or steroid derivative or the "enzyme (variant) for (catalyzing) the C19 hydroxylation of a steroid or steroid derivative" or the like is to be understood as a P450 BM3 monooxygenase (BM3) variant capable of C19-hydroxylation which in turn leads to formation of estradiol or estrone and possibly their derivatives.

In addition, without being bound by theory, for steroids with a **4-en-3-one-A-ring** like (17beta)-17-hydroxyandrost-4-en-3-one (synonym: testosterone) the product of C19-hydroxylation is typically stable and can be isolated. These kind of products can then by further functionalization under dehydrogenation form the same instable intermediate and subsequently under aromatization estradiol and estradiol derivatives (as shown for similar estrone derivatives by (F. Templeton 1997).

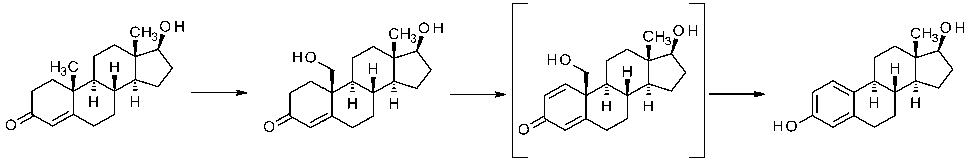

The term **"nucleic acid"** refers to deoxyribonucleotides or ribonucleotides and polymers thereof composed of monomers (nucleotides) containing a sugar, phosphate and a base that is either a purine or pyrimidine. For example and without limitation nucleic acids may occur in single- or double-stranded form. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues.

**"Sequence identity", "percent identity"** or **"percent (%) sequence identity"** describes how similar a query sequence is to a target sequence, more precisely how many characters in each sequence are identical after alignment. Sequence identity can be calculated using BLAST (basic local alignment search tool, NCBI), which performs comparisons between pairs of sequences, searching for regions of local similarity. Suitable alignment methods are known in the art, e.g. Needleman-Wunsch algorithm for global-global alignment, using BLOSUM62 matrix, with gap opening penalty of 11 and a gap extension penalty of 1. Afterwards, the pairs of aligned identical residues can be counted and then divided by the total length of the alignment (including gaps, internal as well as external) to arrive at the percent identity value.

As used herein the term **"specifically hybridize"** or **"selectively hybridize"** refers to a reaction of the nucleic acid sequence in question in a hybridization solution containing 0.5 M sodium phosphate buffer, pH 7.2 containing 7% SDS, 1 mM EDTA and 100 mg/ml of salmon sperm DNA at 65°C for 16 hours and washing twice at 65°C for twenty minutes in a washing solution containing 9.5 x SSC and 0.1% SDS

A **"host cell"** is a cell that is used to receive, maintain, reproduce and amplify a vector. A host cell can also be used to express the polypeptide encoded by the vector. The nucleic acid contained in the vector is replicated when the host cell divides, thereby amplifying the nucleic acids.

The term **"expression system"** refers to any protein production system known in the art and suitable for production of P450-BM3 variants according to what is described herein. Suitable expression systems are well known in the art and example systems comprise *E. coli* strains such as DH5alpha, BL21 (DE3), or Rosetta (DE3), but also various other bacterial and non bacterial systems. Suitable expression systems include gram positive bacteria, such as *Bacillus* such as *Bacillus megaterium* or *Bacillus subtilis, Rhodococcus,* or yeasts expression systems such as *Komagataella phaffii or Saccharomyces cerevisiae* The term **"vector",** as used herein, is known in the art and refers to a nucleic acid molecule capable of propagating a nucleic acid molecule to which it is linked. The term further comprises plasmids (non-viral) and viral vectors.

The **"selectivity"** of a biotransformation process or enzyme can be determined based on the selectivity factor or target product ratio. Other methods to determine selectivity are known to the skilled person. In case of doubt the selectivity factor as defined herein shall control.

The **"selectivity factor"** of a reaction is a measure for the selectivity of the reaction and is calculated by dividing the measured product concentration by the consumed substrate concentration (which is the difference between the initial substrate concentration and the measured substrate concentration after the biotransformation process). Product and substrate concentration can be measured as known in the art and as described in the examples. Usual methods comprise HPLC analyses using substrate and product standards as references and for quantification. If not specified otherwise, the selectivity factor for the C19 hydroxylation of at least one steroid or derivative thereof is calculated based on the steroid or derivative thereof (for the consumed substrate concentration) and the C19 hydroxylated steroid or derivative thereof (for the product concentration). Where the C19 hydroxylated steroid is further converted into a secondary product, selectivity factor may also be calculated with regard to the commercially relevant product.

The **"target product ratio"** (TPR) of a reaction is calculated as integrated area (in HPLC chromatogram) for the desired product divided by the sum of all integrated areas for products. The product may be the C19 hydroxylated steroid. Where the C19 hydroxylated steroid is further converted into a secondary product, target product ratio may also be calculated with regard to the commercially relevant product. For example, with regard to the conversion from ADD to the product estrone, the area under the curve for estrone was divided by the area under the curve for all other products (i.e. not for the educt ADD). In case of doubt, reference is made to the various examples described throughout the disclosure.

The **"product yield"** is calculated by dividing the measured product concentration by the initial substrate concentration in the experiment. The product yield for a given P450-BM3 variant is therefore specific for a given substrate and a given product under given process conditions. For example, the substrate may be a steroid and the product may be the C19 hydroxylated steroid. Where the C19 hydroxylated steroid is further converted into a secondary product, product yield may also be calculated with regard to the commercially relevant product.

The **"product titer"** is provided in mg/L or g/L and is an alternative measure to describe the obtainable yields for a biotransformation product. If not specified elsewhere herein, product titer is determined under standard conditions as described in Example 2.

As used herein the term **"space time yield"** refers to the amount of product generated in a specific volume in a defined time e.g. gram per liter per hour. In some aspects details of a given space time yield are combined with the length of time (duration) for which the reaction proceeds since it is preferred that once a given space time yield has been reached the value does not decrease again. If the space time yield is given as space time yield per day it is given as g/(L*d) where "d" refers to a time span between 22-24 hours.

In some aspects described herein in addition or alternatively to determining the space time yield the ratio of steroid hydroxylated at C19 to steroid to be hydroxylated- i.e. the product yield - is determined and/or the ratio of enzyme to steroid to be hydroxylated is determined. The ratio of enzyme to steroid to be hydroxylated can in some cases be performed via determining the ratio of used amount of cell extract and/or used amount of whole cells to steroid to be hydroxylated. Determining the ratio of enzyme/cell extract/whole cells to steroid to be hydroxylated allows assessing the turnover per enzyme/per enzyme quantity.

As used herein the term **"gene"** means a DNA sequence made up of nucleotides comprising a region (transcribed region), which is transcribed into an RNA molecule (e.g. directly into a mRNA without intron sequences) in a cell, operably linked to regulatory regions capable of regulating the expression of the polypeptide. A gene may thus comprise several operably linked sequences, such as untranslated regulatory regions (e.g. a promoter, enhancer, repressor), a 5' leader sequence comprising e.g. sequences involved in translation initiation, a (protein) coding region (cDNA or genomic DNA) and a 3' non-translated sequence comprising e.g. transcription termination sites.

The term **"plasmid",** as used herein, refers to plasmid vectors, i.e. circular DNA sequence that are capable of autonomous replication within a suitable host due to an origin of replication ("ORI"). Furthermore, a plasmid may comprise a selectable marker to indicate the success of the transformation or other procedures meant to introduce foreign DNA into a cell and a multiple cloning site which includes multiple restriction enzyme consensus sites to enable the insertion of an insert. Plasmid vectors called cloning or donor vectors are used to ease the cloning and to amplify a sequence of interest. Plasmid vectors called expression or acceptor vectors are specifically for the expression of a gene of interest in a defined target cell. Those plasmid vectors generally show an expression cassette, consisting of a promoter, a ribosomal binding site, the transgene and a terminator sequence. For expression control those plasmids contain a repressor, which is localized on the plasmid backbone. Expression plasmids can be shuttle plasmids containing elements that enable the propagation and selection in different host cells.

As used herein the term **"whole cells"** is used synonymously with the term "biomass" and refers to the fact that the P450-monooxygenase is provided in the cells it is expressed in i.e. that the cell walls of said cells were not purposefully disrupted.

### EMBODIMENTS

According to a **first aspect** of what is described a cytochrome P450 BM3 monooxygenase (BM3) variant is provided for catalyzing the C19 hydroxylation of a steroid or steroid derivative wherein the BM3 variant comprises compared to SEQ ID NO 1 at least the mutations D80I and H236I and K440C.

Accordingly, what is described herein relates to a cytochrome P450 BM3 monooxygenase (BM3) variant for catalyzing the C19 hydroxylation of a steroid or steroid derivative wherein the BM3 variant comprises compared to SEQ ID NO 1 the mutations D80I and H236I and K440C.

In a preferred embodiment of the first aspect of what is described a cytochrome P450 BM3 monooxygenase (BM3) variant is provided for catalyzing the C19 hydroxylation of a steroid or steroid derivative having at least 80 % sequence identity with SEQ ID NO 1, wherein the BM3 variant comprises compared to SEQ ID NO 1 at least the mutations D80I and H236I and K440C .

In a preferred embodiment of the first aspect of what is described a cytochrome P450 BM3 monooxygenase (BM3) variant is provided for catalyzing the C19 hydroxylation of a steroid or steroid derivative having at least 90 % sequence identity with SEQ ID NO 1, wherein the BM3 variant comprises compared to SEQ ID NO 1 at least the mutations D80I and H236I and K440C .

In a preferred embodiment of the first aspect of what is described a cytochrome P450 BM3 monooxygenase (BM3) variant is provided for catalyzing the C19 hydroxylation of a steroid or steroid derivative having at least 95 % sequence identity with SEQ ID NO 1, wherein the BM3 variant comprises compared to SEQ ID NO 1 at least the mutations D80I and H236I and K440C .

In a preferred embodiment of the first aspect of what is described a cytochrome P450 BM3 monooxygenase (BM3) variant is provided for catalyzing the C19 hydroxylation of a steroid or steroid derivative having at least 95 % sequence identity with SEQ ID NO 1, wherein the BM3 variant comprises compared to SEQ ID NO 1 at least the mutations D80I and H236I and K440C .

In a preferred embodiment of the first aspect of what is described a cytochrome P450 BM3 monooxygenase (BM3) variant is provided for catalyzing the C19 hydroxylation of a steroid or steroid derivative having at least 98 % sequence identity with SEQ ID NO 1, wherein the BM3 variant comprises compared to SEQ ID NO 1 at least the mutations D80I and H236I and K440C.

In a preferred embodiment of the first aspect of what is described a cytochrome P450 BM3 monooxygenase (BM3) variant is provided for catalyzing the C19 hydroxylation of a steroid or steroid derivative having at least 99 % sequence identity with SEQ ID NO 1, wherein the BM3 variant comprises compared to SEQ ID NO 1 at least the mutations D80I and H236I and K440C.

It was found that the P450 BM3 monooxygenase (BM3) variants having at least 99 % sequence identity with SEQ ID NO 1 and comprising compared to SEQ ID NO 1 at least the mutations D80I and H236I and K440C achieved an improved titer of steroid hydroxylated at C19 compared to SEQ ID NO 1 (cf. Example 3).

In a preferred embodiment of the first aspect the steroid or steroid derivative to be hydroxylated at C19 is a steroid with a 1,4-dien-3-one-A-ring. Thus, the C19-hydroxylation leads to formation of estradiol or estrone and their derivatives.

In an especially preferred embodiment of the first aspect the steroid to be hydroxylated at C19 is androsta-1,4-dien-3,17-dione (ADD) leading to the formation of estrone.

In a preferred embodiment of the first aspect the cytochrome P450 BM3 monooxygenase (BM3) variant comprises compared to SEQ ID NO 1 a combination of mutations selected from the list consisting of SEQ ID NO
2) Q73W, D80I, T175Y, A225V, H236I, G240R, K440C
3) Q73W, D80I, T175Y, A225V, H236I, K440C
4) Q73W, D80I, T175Y, D182H, H236I, G240R, K440C
5) Q73W, D80I, T175Y, D182H, A225V, H236I, G240R, K440C
6) Q73W, D80I, T175Y, D182H, A225V, H236I, K440C
7) Q73W, D80I, T175Y, D182H, H236I, K440C
8) Q73W, D80I, T175Y, D182I, H236I, G240R, K440C
9) Q73W, D80I, T175Y, D182I, A225V, H236I, G240R, K440C
10) Q73W, D80I, T175Y, D182I, A225V, H236I, K440C
11) Q73W, D80I, T175Y, D182I, H236I, K440C
12) Q73W, K76I, D80I, T175Y, H236I, G240R, K440C
13) Q73W, K76I, D80I, T175Y, A225V, H236I, G240R, K440C
14) Q73W, K76I, D80I, T175Y, A225V, H236I, K440C
15) Q73W, K76I, D80I, T175Y, D182H, A225V, H236I, G240R, K440C
16) Q73W, K76I, D80I, T175Y, D182I, H236I, G240R, K440C
17) Q73W, K76I, D80I, T175Y, D182I, A225V, H236I, G240R, K440C
18) Q73W, K76I, D80I, T175Y, R179L, H236I, G240R, K440C
19) Q73W, K76I, D80I, T175Y, R179L, D182I, H236I, G240R, K440C
20) Q73W, K76I, D80I, T175Y, H236I, K440C
21) Q73W, D80I, T175Y, R179L, H236I, G240R, K440C
22) Q73W, D80I, T175Y, R179L, A225V, H236I, G240R, K440C
23) Q73W, D80I, T175Y, R179L, D182H, H236I, G240R, K440C
24) Q73W, D80I, T175Y, R179L, D182H, A225V, H236I, G240R, K440C
25) Q73W, D80I, T175Y, R179L, D182H, H236I, K440C
26) Q73W, D80I, T175Y, R179L, D182I, H236I, G240R, K440C
27) Q73W, D80I, T175Y, R179L, D182I, A225V, H236I, G240R, K440C
28) Q73W, D80I, T175Y, R179L, D182I, A225V, H236I, K440C
29) Q73W, D80I, T175Y, R179L, D182I, H236I, K440C
30) Q73W, D80I, T175Y, R179L, H236I, K440C
31) Q73W, D80I, T175Y, H236I, K440C
32) Q73L, D80I, T175Y, H236I, G240R, K440C
33) Q73L, D80I, T175Y, A225V, H236I, G240R, K440C
34) Q73L, D80I, T175Y, A225V, H236I, K440C
35) Q73L, D80I, T175Y, D182H, H236I, G240R, K440C
36) Q73L, D80I, T175Y, D182I, H236I, G240R, K440C
37) Q73L, K76I, D80I, T175Y, H236I, G240R, K440C
38) Q73L, K76I, D80I, T175Y, A225V, H236I, G240R, K440C
39) Q73L, K76I, D80I, T175Y, R179L, H236I, G240R, K440C
40) Q73L, K76I, D80I, T175Y, R179L, A225V, H236I, G240R, K440C
41) Q73L, K76I, D80I, T175Y, R179L, D182H, A225V, H236I, G240R, K440C
42) Q73L, K76I, D80I, T175Y, H236I, K440C
43) Q73L, D80I, T175Y, R179L, H236I, G240R, K440C
44) Q73L, D80I, T175Y, R179L, A225V, H236I, K440C
45) Q73L, D80I, T175Y, R179L, D182I, A225V, H236I, K440C
46) Q73L, D80I, T175Y, R179L, D182I, H236I, K440C
47) Q73L, D80I, T175Y, R179L, H236I, K440C
48) Q73L, D80I, T175Y, H236I, K440C
49) D80I, F173W, H236I, G240R, T269V, K440C
50) D80I, F173W, T175Y, D182I, H236I, K440C
51) D80I, F173W, T175Y, H236I, K440C
52) D80I, F173W, T175Y, R179L, D182H, H236I, T269V, K440C
53) D80I, H236I, K440C
54) D80I, H236I, T269V, K440C
55) D80I, P172G, F173W, R179L, H236I, G240R, T269V, K440C
56) D80I, P172G, T175Y, R179L, D182I, H236I, G240R, K440C
57) D80I, R179L, D182H, H236I, T269V, K440C
58) D80I, R179L, D182I, H236I, T269V, K440C
59) D80I, R179L, H236I, K440C
60) D80I, T175Y, A225V, H236I, G240R, T269V, K440C
61) D80I, T175Y, H236I, G240R, K440C
62) K76I, D80I, F173W, T175Y, H236I, K440C
63) K76I, D80I, N186V, A225V, H236I, K440C
64) K76I, D80I, N186V, H236I, K440C
65) K76I, D80I, R179L, H236I, G240R, K440C
66) K76I, D80I, R179L, H236I, K440C
67) K76I, D80I, R179L, N186V, H236I, K440C
68) Q73W, D80I, T175Y, H236I, G240R, K440C
69) Q73W, K76I, D80I, T175Y, D182H, H236I, G240R, K440C
70) Q73W, K76I, D80I, T175Y, D182H, N186V, A225V, H236I, G240R, K440C
71) Q73W, K76I, D80I, T175Y, D182H, N186V, H236I, G240R, K440C
72) Q73W, K76I, D80I, T175Y, D182I, A225V, H236I, K440C
73) Q73W, K76I, D80I, T175Y, D182I, H236I, K440C
74) Q73W, K76I, D80I, T175Y, R179L, D182H, H236I, G240R, K440C
75) Q73L, D80I, T175Y, R179L, H236I, G240R, T269V, K440C
as well as possible further mutations.

In a preferred embodiment of the first aspect the cytochrome P450 BM3 monooxygenase (BM3) variant comprises compared to SEQ ID NO 1 a combination of mutations selected from the list consisting of SEQ ID NO
2) Q73W, D80I, T175Y, A225V, H236I, G240R, K440C
3) Q73W, D80I, T175Y, A225V, H236I, K440C
4) Q73W, D80I, T175Y, D182H, H236I, G240R, K440C
5) Q73W, D80I, T175Y, D182H, A225V, H236I, G240R, K440C
6) Q73W, D80I, T175Y, D182H, A225V, H236I, K440C
7) Q73W, D80I, T175Y, D182H, H236I, K440C
8) Q73W, D80I, T175Y, D182I, H236I, G240R, K440C
9) Q73W, D80I, T175Y, D182I, A225V, H236I, G240R, K440C
10) Q73W, D80I, T175Y, D182I, A225V, H236I, K440C
11) Q73W, D80I, T175Y, D182I, H236I, K440C
12) Q73W, K76I, D80I, T175Y, H236I, G240R, K440C
13) Q73W, K76I, D80I, T175Y, A225V, H236I, G240R, K440C
14) Q73W, K76I, D80I, T175Y, A225V, H236I, K440C
15) Q73W, K76I, D80I, T175Y, D182H, A225V, H236I, G240R, K440C
16) Q73W, K76I, D80I, T175Y, D182I, H236I, G240R, K440C
17) Q73W, K76I, D80I, T175Y, D182I, A225V, H236I, G240R, K440C
18) Q73W, K76I, D80I, T175Y, R179L, H236I, G240R, K440C
19) Q73W, K76I, D80I, T175Y, R179L, D182I, H236I, G240R, K440C
20) Q73W, K76I, D80I, T175Y, H236I, K440C
21) Q73W, D80I, T175Y, R179L, H236I, G240R, K440C
22) Q73W, D80I, T175Y, R179L, A225V, H236I, G240R, K440C
23) Q73W, D80I, T175Y, R179L, D182H, H236I, G240R, K440C
24) Q73W, D80I, T175Y, R179L, D182H, A225V, H236I, G240R, K440C
25) Q73W, D80I, T175Y, R179L, D182H, H236I, K440C
26) Q73W, D80I, T175Y, R179L, D182I, H236I, G240R, K440C
27) Q73W, D80I, T175Y, R179L, D182I, A225V, H236I, G240R, K440C
28) Q73W, D80I, T175Y, R179L, D182I, A225V, H236I, K440C
29) Q73W, D80I, T175Y, R179L, D182I, H236I, K440C
30) Q73W, D80I, T175Y, R179L, H236I, K440C
31) Q73W, D80I, T175Y, H236I, K440C
32) Q73L, D80I, T175Y, H236I, G240R, K440C
33) Q73L, D80I, T175Y, A225V, H236I, G240R, K440C
34) Q73L, D80I, T175Y, A225V, H236I, K440C
35) Q73L, D80I, T175Y, D182H, H236I, G240R, K440C
36) Q73L, D80I, T175Y, D182I, H236I, G240R, K440C
37) Q73L, K76I, D80I, T175Y, H236I, G240R, K440C
38) Q73L, K76I, D80I, T175Y, A225V, H236I, G240R, K440C
39) Q73L, K76I, D80I, T175Y, R179L, H236I, G240R, K440C
40) Q73L, K76I, D80I, T175Y, R179L, A225V, H236I, G240R, K440C
41) Q73L, K76I, D80I, T175Y, R179L, D182H, A225V, H236I, G240R, K440C
42) Q73L, K76I, D80I, T175Y, H236I, K440C
43) Q73L, D80I, T175Y, R179L, H236I, G240R, K440C
44) Q73L, D80I, T175Y, R179L, A225V, H236I, K440C
45) Q73L, D80I, T175Y, R179L, D182I, A225V, H236I, K440C
46) Q73L, D80I, T175Y, R179L, D182I, H236I, K440C
47) Q73L, D80I, T175Y, R179L, H236I, K440C
48) Q73L, D80I, T175Y, H236I, K440C
49) D80I, F173W, H236I, G240R, T269V, K440C
50) D80I, F173W, T175Y, D182I, H236I, K440C
51) D80I, F173W, T175Y, H236I, K440C
52) D80I, F173W, T175Y, R179L, D182H, H236I, T269V, K440C
53) D80I, H236I, K440C
54) D80I, H236I, T269V, K440C
55) D80I, P172G, F173W, R179L, H236I, G240R, T269V, K440C
56) D80I, P172G, T175Y, R179L, D182I, H236I, G240R, K440C
57) D80I, R179L, D182H, H236I, T269V, K440C
58) D80I, R179L, D182I, H236I, T269V, K440C
59) D80I, R179L, H236I, K440C
60) D80I, T175Y, A225V, H236I, G240R, T269V, K440C
61) D80I, T175Y, H236I, G240R, K440C
62) K76I, D80I, F173W, T175Y, H236I, K440C
63) K76I, D80I, N186V, A225V, H236I, K440C
64) K76I, D80I, N186V, H236I, K440C
65) K76I, D80I, R179L, H236I, G240R, K440C
66) K76I, D80I, R179L, H236I, K440C
67) K76I, D80I, R179L, N186V, H236I, K440C
68) Q73W, D80I, T175Y, H236I, G240R, K440C
69) Q73W, K76I, D80I, T175Y, D182H, H236I, G240R, K440C
70) Q73W, K76I, D80I, T175Y, D182H, N186V, A225V, H236I, G240R, K440C
71) Q73W, K76I, D80I, T175Y, D182H, N186V, H236I, G240R, K440C
72) Q73W, K76I, D80I, T175Y, D182I, A225V, H236I, K440C
73) Q73W, K76I, D80I, T175Y, D182I, H236I, K440C
74) Q73W, K76I, D80I, T175Y, R179L, D182H, H236I, G240R, K440C
75) Q73L, D80I, T175Y, R179L, H236I, G240R, T269V, K440C.

It was surprisingly found that all variants comprising mutations D80I and H236I and K440C showed an improved estrone titer i.e. an increased amount of steroid hydroxylated at C19 whereas with other variants this was not necessarily the case (cf. example 3).

In an especially preferred embodiment of the first aspect the steroid to be hydroxylated at C19 is androsta-1,4-dien-3,17-dione (ADD) leading to the formation of estrone and the cytochrome P450 BM3 monooxygenase (BM3) variant comprises compared to SEQ ID NO 1 a combination of mutations selected from the list consisting of SEQ ID NO
2) Q73W, D80I, T175Y, A225V, H236I, G240R, K440C
3) Q73W, D80I, T175Y, A225V, H236I, K440C
4) Q73W, D80I, T175Y, D182H, H236I, G240R, K440C
5) Q73W, D80I, T175Y, D182H, A225V, H236I, G240R, K440C
6) Q73W, D80I, T175Y, D182H, A225V, H236I, K440C
7) Q73W, D80I, T175Y, D182H, H236I, K440C
8) Q73W, D80I, T175Y, D182I, H236I, G240R, K440C
9) Q73W, D80I, T175Y, D182I, A225V, H236I, G240R, K440C
10) Q73W, D80I, T175Y, D182I, A225V, H236I, K440C
11) Q73W, D80I, T175Y, D182I, H236I, K440C
12) Q73W, K76I, D80I, T175Y, H236I, G240R, K440C
13) Q73W, K76I, D80I, T175Y, A225V, H236I, G240R, K440C
14) Q73W, K76I, D80I, T175Y, A225V, H236I, K440C
15) Q73W, K76I, D80I, T175Y, D182H, A225V, H236I, G240R, K440C
16) Q73W, K76I, D80I, T175Y, D182I, H236I, G240R, K440C
17) Q73W, K76I, D80I, T175Y, D182I, A225V, H236I, G240R, K440C
18) Q73W, K76I, D80I, T175Y, R179L, H236I, G240R, K440C
19) Q73W, K76I, D80I, T175Y, R179L, D182I, H236I, G240R, K440C
20) Q73W, K76I, D80I, T175Y, H236I, K440C
21) Q73W, D80I, T175Y, R179L, H236I, G240R, K440C
22) Q73W, D80I, T175Y, R179L, A225V, H236I, G240R, K440C
23) Q73W, D80I, T175Y, R179L, D182H, H236I, G240R, K440C
24) Q73W, D80I, T175Y, R179L, D182H, A225V, H236I, G240R, K440C
25) Q73W, D80I, T175Y, R179L, D182H, H236I, K440C
26) Q73W, D80I, T175Y, R179L, D182I, H236I, G240R, K440C
27) Q73W, D80I, T175Y, R179L, D182I, A225V, H236I, G240R, K440C
28) Q73W, D80I, T175Y, R179L, D182I, A225V, H236I, K440C
29) Q73W, D80I, T175Y, R179L, D182I, H236I, K440C
30) Q73W, D80I, T175Y, R179L, H236I, K440C
31) Q73W, D80I, T175Y, H236I, K440C
32) Q73L, D80I, T175Y, H236I, G240R, K440C
33) Q73L, D80I, T175Y, A225V, H236I, G240R, K440C
34) Q73L, D80I, T175Y, A225V, H236I, K440C
35) Q73L, D80I, T175Y, D182H, H236I, G240R, K440C
36) Q73L, D80I, T175Y, D182I, H236I, G240R, K440C
37) Q73L, K76I, D80I, T175Y, H236I, G240R, K440C
38) Q73L, K76I, D80I, T175Y, A225V, H236I, G240R, K440C
39) Q73L, K76I, D80I, T175Y, R179L, H236I, G240R, K440C
40) Q73L, K76I, D80I, T175Y, R179L, A225V, H236I, G240R, K440C
41) Q73L, K76I, D80I, T175Y, R179L, D182H, A225V, H236I, G240R, K440C
42) Q73L, K76I, D80I, T175Y, H236I, K440C
43) Q73L, D80I, T175Y, R179L, H236I, G240R, K440C
44) Q73L, D80I, T175Y, R179L, A225V, H236I, K440C
45) Q73L, D80I, T175Y, R179L, D182I, A225V, H236I, K440C
46) Q73L, D80I, T175Y, R179L, D182I, H236I, K440C
47) Q73L, D80I, T175Y, R179L, H236I, K440C
48) Q73L, D80I, T175Y, H236I, K440C
49) D80I, F173W, H236I, G240R, T269V, K440C
50) D80I, F173W, T175Y, D182I, H236I, K440C
51) D80I, F173W, T175Y, H236I, K440C
52) D80I, F173W, T175Y, R179L, D182H, H236I, T269V, K440C
53) D80I, H236I, K440C
54) D80I, H236I, T269V, K440C
55) D80I, P172G, F173W, R179L, H236I, G240R, T269V, K440C
56) D80I, P172G, T175Y, R179L, D182I, H236I, G240R, K440C
57) D80I, R179L, D182H, H236I, T269V, K440C
58) D80I, R179L, D182I, H236I, T269V, K440C
59) D80I, R179L, H236I, K440C
60) D80I, T175Y, A225V, H236I, G240R, T269V, K440C
61) D80I, T175Y, H236I, G240R, K440C
62) K76I, D80I, F173W, T175Y, H236I, K440C
63) K76I, D80I, N186V, A225V, H236I, K440C
64) K76I, D80I, N186V, H236I, K440C
65) K76I, D80I, R179L, H236I, G240R, K440C
66) K76I, D80I, R179L, H236I, K440C
67) K76I, D80I, R179L, N186V, H236I, K440C
68) Q73W, D80I, T175Y, H236I, G240R, K440C
69) Q73W, K76I, D80I, T175Y, D182H, H236I, G240R, K440C
70) Q73W, K76I, D80I, T175Y, D182H, N186V, A225V, H236I, G240R, K440C
71) Q73W, K76I, D80I, T175Y, D182H, N186V, H236I, G240R, K440C
72) Q73W, K76I, D80I, T175Y, D182I, A225V, H236I, K440C
73) Q73W, K76I, D80I, T175Y, D182I, H236I, K440C
74) Q73W, K76I, D80I, T175Y, R179L, D182H, H236I, G240R, K440C
75) Q73L, D80I, T175Y, R179L, H236I, G240R, T269V, K440C.

In another preferred embodiment of the first aspect the cytochrome P450 BM3 monooxygenase (BM3) variant comprises compared to SEQ ID NO 1 the further mutations Q73W, T175Y and G240R and is selected from the list consisting of SEQ ID NO
26) Q73W, D80I, T175Y, R179L, D182I, H236I, G240R, K440C
74) Q73W, K76I, D80I, T175Y, R179L, D182H, H236I, G240R, K440C
17) Q73W, K76I, D80I, T175Y, D182I, A225V, H236I, G240R, K440C
68) Q73W, D80I, T175Y, H236I, G240R, K440C
2) Q73W, D80I, T175Y, A225V, H236I, G240R, K440C
4) Q73W, D80I, T175Y, D182H, H236I, G240R, K440C
8) Q73W, D80I, T175Y, D182I, H236I, G240R, K440C
12) Q73W, K76I, D80I, T175Y, H236I, G240R, K440C.

These variants all show an especially improved estrone titer i.e. an increased amount of steroid hydroxylated at C19 compared to SEQ ID NO 1 (cf. Table 2).

Hence in another preferred embodiment of the first aspect the cytochrome P450 BM3 monooxygenase (BM3) variant has at least 99 % sequence identity with SEQ ID NO 1 and comprises compared to SEQ ID NO 1 the further mutations Q73W, T175Y and G240R and is selected from the list consisting of SEQ ID NO
26) Q73W, D80I, T175Y, R179L, D182I, H236I, G240R, K440C
74) Q73W, K76I, D80I, T175Y, R179L, D182H, H236I, G240R, K440C
17) Q73W, K76I, D80I, T175Y, D182I, A225V, H236I, G240R, K440C
68) Q73W, D80I, T175Y, H236I, G240R, K440C
2) Q73W, D80I, T175Y, A225V, H236I, G240R, K440C
4) Q73W, D80I, T175Y, D182H, H236I, G240R, K440C
8) Q73W, D80I, T175Y, D182I, H236I, G240R, K440C
12) Q73W, K76I, D80I, T175Y, H236I, G240R, K440C.

In an especially preferred embodiment of the first aspect the steroid to be hydroxylated at C19 is androsta-1,4-dien-3,17-dione (ADD) leading to the formation of estrone and the cytochrome P450 BM3 monooxygenase (BM3) variant comprises compared to SEQ ID NO 1 the further mutations Q73W, T175Y and G240R and is selected from the list consisting of SEQ ID NO
26) Q73W, D80I, T175Y, R179L, D182I, H236I, G240R, K440C
74) Q73W, K76I, D80I, T175Y, R179L, D182H, H236I, G240R, K440C
17) Q73W, K76I, D80I, T175Y, D182I, A225V, H236I, G240R, K440C
68) Q73W, D80I, T175Y, H236I, G240R, K440C
2) Q73W, D80I, T175Y, A225V, H236I, G240R, K440C
4) Q73W, D80I, T175Y, D182H, H236I, G240R, K440C
8) Q73W, D80I, T175Y, D182I, H236I, G240R, K440C
12) Q73W, K76I, D80I, T175Y, H236I, G240R, K440C.

Thus, in another especially preferred embodiment of the first aspect the steroid to be hydroxylated at C19 is androsta-1,4-dien-3,17-dione (ADD) leading to the formation of estrone and the cytochrome P450 BM3 monooxygenase (BM3) variant has at least 99 % sequence identity with SEQ ID NO 1 and comprises compared to SEQ ID NO 1 the further mutations Q73W, T175Y and G240R and is selected from the list consisting of SEQ ID NO
SEQ ID NO 26) Q73W, D80I, T175Y, R179L, D182I, H236I, G240R, K440C
SEQ ID NO 74) Q73W, K76I, D80I, T175Y, R179L, D182H, H236I, G240R, K440C
SEQ ID NO 17) Q73W, K76I, D80I, T175Y, D182I, A225V, H236I, G240R, K440C
SEQ ID NO 68) Q73W, D80I, T175Y, H236I, G240R, K440C
SEQ ID NO 2) Q73W, D80I, T175Y, A225V, H236I, G240R, K440C
SEQ ID NO 4) Q73W, D80I, T175Y, D182H, H236I, G240R, K440C
SEQ ID NO 8) Q73W, D80I, T175Y, D182I, H236I, G240R, K440C
SEQ ID NO 12) Q73W, K76I, D80I, T175Y, H236I, G240R, K440C.

In an alternative preferred embodiment of the first aspect the cytochrome P450 BM3 monooxygenase (BM3) variant comprises compared to SEQ ID NO 1 the further mutations Q73L, T175Y and G240R and is selected from the list consisting of SEQ ID NO
38) Q73L, K76I, D80I, T175Y, A225V, H236I, G240R, K440C
36) Q73L, D80I, T175Y, D182I, H236I, G240R, K440C
35) Q73L, D80I, T175Y, D182H, H236I, G240R, K440C
45) Q73L, D80I, T175Y, R179L, D182I, A225V, H236I, K440C
48) Q73L, D80I, T175Y, H236I, K440C.

Therefore, in another alternative preferred embodiment of the first aspect the cytochrome P450 BM3 monooxygenase (BM3) variant has at least 99 % sequence identity with SEQ ID NO 1 and comprises compared to SEQ ID NO 1 the further mutations Q73L, T175Y and G240R and is selected from the list consisting of (SEQ ID NO)
SEQ ID NO 38) Q73L, K76I, D80I, T175Y, A225V, H236I, G240R, K440C
SEQ ID NO 36) Q73L, D80I, T175Y, D182I, H236I, G240R, K440C
SEQ ID NO 35) Q73L, D80I, T175Y, D182H, H236I, G240R, K440C
SEQ ID NO 45) Q73L, D80I, T175Y, R179L, D182I, A225V, H236I, K440C
SEQ ID NO 48) Q73L, D80I, T175Y, H236I, K440C.

These variants all show an especially improved estrone titer i.e. an increased amount of steroid hydroxylated at C19 compared to SEQ ID NO 1 (cf. Table 2).

In an especially preferred embodiment of the first aspect the steroid to be hydroxylated at C19 is androsta-1,4-dien-3,17-dione (ADD) leading to the formation of estrone and the cytochrome P450 BM3 monooxygenase (BM3) variant comprises compared to SEQ ID NO 1 the further mutations Q73L, T175Y and G240R and is selected from the list consisting of SEQ ID NO
38) Q73L, K76I, D80I, T175Y, A225V, H236I, G240R, K440C
36) Q73L, D80I, T175Y, D182I, H236I, G240R, K440C
35) Q73L, D80I, T175Y, D182H, H236I, G240R, K440C
45) Q73L, D80I, T175Y, R179L, D182I, A225V, H236I, K440C
48) Q73L, D80I, T175Y, H236I, K440C.

Hence, in another especially preferred embodiment of the first aspect the steroid to be hydroxylated at C19 is androsta-1,4-dien-3,17-dione (ADD) leading to the formation of estrone and the cytochrome P450 BM3 monooxygenase (BM3) variant has at least 99% sequence identity with SEQ ID NO 1 and comprises compared to SEQ ID NO 1 the further mutations Q73L, T175Y and G240R and is selected from the list consisting of (SEQ ID NO)
SEQ ID NO 38) Q73L, K76I, D80I, T175Y, A225V, H236I, G240R, K440C
SEQ ID NO 36) Q73L, D80I, T175Y, D182I, H236I, G240R, K440C
SEQ ID NO 35) Q73L, D80I, T175Y, D182H, H236I, G240R, K440C
SEQ ID NO 45) Q73L, D80I, T175Y, R179L, D182I, A225V, H236I, K440C
SEQ ID NO 48) Q73L, D80I, T175Y, H236I, K440C.

In a **second aspect** what is described herein relates to a method (i.e. an in vitro method) for generating a steroid hydroxylated at C19 using a cytochrome P450 BM3 monooxygenase (BM3) variant of the first aspect wherein the generated steroid hydroxylated at C19 is present in a range of greater than 0,4 g/(L*d) preferably in the range of 0,4 g/(L*d) to 50 g/(L*d) .

In a preferred embodiment of the second aspect the generated steroid hydroxylated at C19 is present in a range of 0,4 g/(L*d) to 8 g/(L*d). As shown for instance in examples 4.4 and 4.6 this range was achieved.

In a preferred embodiment of the second aspect the generated steroid hydroxylated at C19 is present in a range of 4 g/L to 8 g/L. As shown in example 4.4 this range was achieved.

In one embodiment the substrate is added in a micronized form. Alternatively, the substrate is added in a non-micronized format or in both formats micronized and non-micronized.

In a preferred embodiment of the second aspect the steroid to be hydroxylated at C19 is testosterone, delta-1-testosterone or androsta-1,4-dien-3,17-dione.

In an especially preferred embodiment of the second aspect the steroid to be hydroxylated at C19 is androsta-1,4-dien-3,17-dione (ADD).

In an especially preferred embodiment of the method (i.e. the in vitro method) of the second aspect the steroid to be hydroxylated at C19 is androsta-1,4-dien-3,17-dione (ADD) leading to the formation of estrone in a range of above 0,4 g/(L*d) , preferably 0,4 g/(L*d) to 50 g/(L*d) .

In another especially preferred embodiment of the method (i.e. the in vitro method) of the second aspect the steroid to be hydroxylated at C19 is androsta-1,4-dien-3,17-dione (ADD) leading to the formation of estrone in a range of 0,4 g/(L*d) to 8 g/(L*d).

In a further especially preferred embodiment of the method (i.e. the in vitro method) of the second aspect the steroid to be hydroxylated at C19 is androsta-1,4-dien-3,17-dione (ADD) leading to the formation of estrone in a range of 4 g/L to 8 g/L.

In a further embodiment of the second aspect the C19 hydroxylation is achieved in the same cells that generated the P450 BM3-monooxygenase.

This has the advantage that no purification of the cytochrome P450 BM3 monooxygenase has to take place thereby saving time and costs.

In a further embodiment of the second aspect the ratio of steroid to be hydroxylated at C19 to steroid hydroxylated at C19 is in the range of 1:4,3 to 1:6. Hence in a preferred embodiment of the method (i.e. the in vitro method) of the second aspect the ratio of androsta-1,4-dien-3,17-dione (ADD) to estrone is in the range of 1:4,3 to 1:6.

In a preferred embodiment the ratio of steroid to be hydroxylated at C19 to steroid hydroxylated at C19 is in the range of 1:4,3 to 1:1. Hence in a preferred embodiment of the method (i.e. the in vitro method) of the second aspect the ratio of androsta-1,4-dien-3,17-dione (ADD) to estrone is in the range of 1:4,3 to 1:1.

Thus, the method (i.e. the in vitro method) of the second aspects achieves high amounts of product.

In a further embodiment of the second aspect the ratio of biomass comprising the P450 BM3 monooxygenase to steroid to be hydroxylated is in the range of 50:1 to 15:1. In a preferred embodiment the ratio of biomass comprising the P450 BM3 monooxygenase to steroid to be hydroxylated is 25:1. Thus, the method (i.e. the in vitro method) of the second aspects achieves a high turnover of substrate per unit of enzyme/unit of biomass. Hence in a preferred embodiment of the method (i.e. the in vitro method) of the second aspect the ratio of biomass comprising the P450 BM3 monooxygenase to androsta-1,4-dien-3,17-dione (ADD) is in the range of 50:1 to 15:1, preferably it is 25:1

In a further embodiment of the second aspect the C19 hydroxylation is achieved in the same cells that generated the P450 BM3-monooxygenase and prior to C19 hydroxylation in said cells 5-Aminolevulinic acid is added to the cell culture medium.

As demonstrated in example 4.6 it was surprisingly found that the addition of 5-Aminolevulinic acid increased the space time yield of the steroid hydroxylated at C19 as well as the estrone titer. In an especially preferred embodiment of this embodiment of the method (i.e. the in vitro method) of the second aspect the steroid to be hydroxylated at C19 is androsta-1,4-dien-3,17-dione (ADD) leading to the formation of estrone.

Hence, in a preferred embodiment of the second aspect the C19 hydroxylation is achieved in the same cells that generated the P450 BM3-monooxygenase and prior to C19 hydroxylation in said cells 5-Aminolevulinic acid is added to the cell culture medium and the generated steroid hydroxylated at C19 is present in a range of 0,4 g/(L*d) to 8 g/(L*d). As shown in example 4.6 this value was surprisingly achieved. In an especially preferred embodiment of this embodiment of the method (i.e. the in vitro method) of the second aspect the steroid to be hydroxylated at C19 is androsta-1,4-dien-3,17-dione (ADD) leading to the formation of estrone.

Moreover, in a preferred embodiment of the second aspect the C19 hydroxylation is achieved in the same cells that generated the P450 BM3-monooxygenase and prior to C19 hydroxylation in said cells 5-Aminolevulinic acid is added to the cell culture medium and the generated steroid hydroxylated at C19 is present in a range of 4 g/L to 8 g/L. As shown in example 4.6 this value was surprisingly achieved. In an especially preferred embodiment of this embodiment of the method (i.e. the in vitro method) of the second aspect the steroid to be hydroxylated at C19 is androsta-1,4-dien-3,17-dione (ADD) leading to the formation of estrone.

In a preferred embodiment of this embodiment the amount of 5-Aminolevulinic acid added to the cell culture medium is in the range of 10 mg/L and 600 mg/L.

In a further embodiment of the second aspect the C19 hydroxylation is achieved in the same cells that generated the P450 BM3-monooxygenase and prior to C19 hydroxylation in said cells toluene in the range of 0,2% (v/v) to 2 % (v/v) is added to the cell culture medium. In an especially preferred embodiment of this embodiment of the method (i.e. the in vitro method) of the second aspect the steroid to be hydroxylated at C19 is androsta-1,4-dien-3,17-dione (ADD) leading to the formation of estrone.

As demonstrated in example 4.2 it was surprisingly found that the addition of toluene increased the space time yield of the steroid hydroxylated at C19.

In a further embodiment of the second aspect the C19 hydroxylation is achieved in the same cells that generated the P450 BM3-monooxygenase and the C19 hydroxylation is carried out in the presence of FDH or GDH and/or wherein the C19 hydroxylation is carried out in the presence of GSH.

As demonstrated in example 4.4 and 4.5 it was surprisingly found that C19 hydroxylation in the presence of FDH or GDH increased the titer of the steroid hydroxylated at C19. In an especially preferred embodiment of this embodiment of the method (i.e. the in vitro method) of the second aspect the steroid to be hydroxylated at C19 is androsta-1,4-dien-3,17-dione (ADD) leading to the formation of estrone.

As demonstrated in example 4.3 it was surprisingly found that C19 hydroxylation in the presence of GSH increased the titer of the steroid hydroxylated at C19. In an especially preferred embodiment of this embodiment of the method (i.e. the in vitro method) of the second aspect the steroid to be hydroxylated at C19 is androsta-1,4-dien-3,17-dione (ADD) leading to the formation of estrone.

In a preferred embodiment of the second aspect where the C19 hydroxylation is achieved in the same cells that generated the P450 BM3-monooxygenase and the C19 hydroxylation is carried out in the presence of FDH or GDH and/or wherein the C19 hydroxylation is carried out in the presence of GSH, the FDH is expressed intracellular, the GDH is present in a range of 0-1 g/L and/or the GSH is present in the range of 0-50 mM.

In a preferred embodiment of the second aspect the C19 hydroxylation is achieved in the same cells that generated the P450 BM3-monooxygenase and the C19 hydroxylation is carried out in the presence of FDH and the FDH is expressed intracellularly and the generated steroid hydroxylated at C19 is present in a range of 4 g/L to 8 g/L. As shown in example 4.4 this range was surprisingly achieved.

In a preferred embodiment of the second aspect the C19 hydroxylation is achieved in the same cells that generated the P450 BM3-monooxygenase and the C19 hydroxylation is carried out in the presence of FDH and the FDH is expressed intracellularly and the generated steroid hydroxylated at C19 is present in a range of 4 g/(L*d) to 8 g/(L*d). As shown in example 4.4 this range was surprisingly achieved.

In a further embodiment of the second aspect the steroid hydroxylated at C19 is purified following the C19 hydroxylation.

In a further embodiment of the second aspect the medium used to hydroxylate the steroid is different from the medium used to generate the P450 BM3-monooxygenase. In one example of this embodiment the medium used to hydroxylate the steroid consists of "old" vs "new" medium in the range of 50%:50% to 0%:100% i.e. at least some of the medium is exchanged prior to starting the hydroxylation. In one example of this embodiment following generation of the P450 BM3-monooxygenase cells are separated from the fermentation medium - for instance via centrifugation - and subsequently resuspended in new fermentation medium at concentration of 200 g/L and then diluted to reach a final concentration of 50 g/L biomass before starting the biotransformation, wherein the dilution is conducted with fermentation medium and/or medium used for biotransformation depending on the ratio of old" vs "new" medium that is to be achieved. In an especially preferred embodiment of this embodiment of the method (i.e. the in vitro method) of the second aspect the steroid to be hydroxylated at C19 is androsta-1,4-dien-3,17-dione (ADD) leading to the formation of estrone.

In a further embodiment of the second aspect prior to C19 hydroxylation in said cells the cells are frozen. In an especially preferred embodiment of this embodiment of the method (i.e. the in vitro method) of the second aspect the steroid to be hydroxylated at C19 is androsta-1,4-dien-3,17-dione (ADD) leading to the formation of estrone.

In a further embodiment of the second aspect the FDH or GDH is provided as lyophilized powder or as fresh biomass or is co-expressed in the cells.

In a preferred embodiment of the second aspect the FDH is co-expressed in the cells, 5-Aminolevulinic acid is present in the cell culture medium at around 300 mg/L and the cells are used as fresh biomass for C19 hydroxylation. In an especially preferred embodiment of this embodiment of the method (i.e. the in vitro method) of the second aspect the steroid to be hydroxylated at C19 is androsta-1,4-dien-3,17-dione (ADD) leading to the formation of estrone.

According to a third aspect of what is described herein, there is provided a nucleic acid encoding for a Cytochrome P450 BM3 monooxygenase variant as described herein.

### EXAMPLES

If not stated otherwise, culture media were prepared in demineralized water and sterilized at 121 °C for 20 minutes or sterile filtered. All other solutions were prepared with MilliQ water and sterile filtered, unless otherwise described. All preparations were performed under sterile conditions.

List of abbreviations: Δ...deletion from amino acid; DMSO...Dimethyl sulfoxide; DMF...Dimethylformamide; EDTA...Ethylendiamintetraacetate; × g...times gravity; IPTG... isopropyl β-D-thiogalactopyranoside; LB... Luria-Bertani broth; OD550... optical density at 550 nm; rpm...revolutions per minute; Pluronic^{®} PE 8100... Pluronic PE types are low-foaming, nonionic surfactants. They are block copolymers in which the central polypropylene glycol group is flanked by two polyethylene glycol groups. PE 8100 conforms to the following structural formula: HO(CH₂CH₂O)ₓ(CH₂C(CH₃)HO)_{y}(CH₂CH₂O)_{z}H. PE 8100 is a polypropylene glycol block copolymer with a molar mass of 2300 g/mol and 10 % polypropylene glycol in the molecule.

Moreover, even though in the following examples the P450 BM3-monooxygenases were provided in the cells they were expressed i.e. in whole cells/as biomass the enzymes can also be used following isolation from the biomass (data not shown).

Furthermore, upstream of the enzyme sequences of Table 1 the His Tag of SEQ ID NO 93 was located except if indicated otherwise.

### Example 1 Cloning of P450 BM3 (BM3) variants

Nucleotide sequences encoding P450-BM3 variants as described herein can be synthesized as known in the art, e.g. as offered by respective service providers such as Eurofins Genomics GmbH (Eurofins Genomics GmbH, Anzinger Str. 7a, 85560 Ebersberg, Germany). In brief, nucleic acid sequences encoding P450-BM3 WT (SEQ ID NO 77) or other P450-BM3 variants as described herein were cloned into an expression vector based on the vector pETM-11 (EMBL Vector Collection, Germany) or another suitable vector. For a detailed description see section 1.2 *"Cloning of cyp102A1 and pETM11-BM3 construction"* of Supporting Information of (Acevedo-Rocha 2018). Genetic elements were introduced into the modified pETM-11 vector by means of commonly known methods. For expression of BM3 variants, the expression vector was introduced into *Escherichia coli* strain BL21 Star^{™} (DE3) (Invitrogen^{™}, Lifetechnologies) cells or *E. coli* BL 21 (DE3) cells.

### Generation of enzyme variants

Nucleotide substitutions (replacements) were introduced into the nucleic acid parent sequence i.e. SEQ ID NO 1. Several Molecular Biological methods can be used to achieve these replacements such as site-directed mutagenesis on codons encoding one or more amino acids, which are selected in advance. The methods for obtaining these site-directed mutations are well known to the skilled person and widely described in the literature (in particular: Directed Mutagenesis: A Practical Approach, 1991, Edited by M.J. McPHERSON, IRL PRESS), or are methods for which it is possible to employ commercial kits (for example the QUIKCHANGE^{™} lightening mutagenesis kit from Qiagen or Stratagene). After site-directed mutagenesis, nucleic acids were transformed into the *Escherichia coli* strain BL21 Star^{™}(DE3) (Invitrogen^{™}, Lifetechnologies).

Transformed cells were tested in order to determine product titer and TPR. Appropriate biotransformation reactions are described below, see e.g. Example 2. Sequence verification was performed as known in the art. Glycerol stocks of the *E. coli* cultures transformed with the respective expression plasmids were prepared by adding one volume of 40% glycerol solution to one volume of *E. coli* culture. For isolation of single bacterial colonies, appropriate dilutions of *E. coli* cultures were plated onto LB-Agar plates containing suitable concentrations of kanamycin and incubated at 37°C until single colonies were obtained.

### Example 2 Cell culture Biotransformation and Analysis

### 2.1 Culture conditions for screening enzyme variants

For screening purposes *E. coli* strain BL21 Star^{™} (DE3) (Invitrogen^{™}, Lifetechnologies) was used as host for expression plasmids (Example 1). For pre-cultures sterilized 2.5-mL 96-well deep-well plates (850301, HJ-Bioanalytik, Erkelenz, Germany) were filled with 490 µL LB-medium (LB Broth Miller, Fisher Bioreagents, BP1426-500) supplemented with kanamycin (Sigma-Aldrich, 50 µg/mL), inoculated with 10 µL of glycerol stocks of the respective variants (that were prepared in 96-well microtiter plates) or alternatively 500 µL LB-medium were inoculated with cell material from Agar plate colonies. Pre-cultures were incubated for 17 hours at 37°C and 250 rpm in a climo shaker ISF1-X (Kühner AG, Birsfelden, Switzerland).

Medium for expression cultures contained Tryptone (12 g/L), Yeast extract (24 g/L), Meat Peptone, tryptically digested (2 g/L), KH2PO4 (2.2 g/L), K2HPO4 (9.4 g/L), glycerol (0.4% [v/v]), Riboflavin (0.001 g/L), Thiamine hydrochloride (0.337 g/L) and Oxford trace element solution (250 µL/L). Tryptone, Yeast extract and Meat peptone were prepared as 50 g/L stock solutions, and K-PO4 was prepared as a 50x stock solution. These stock solutions were sterilized at 121 °C for 20 minutes. Riboflavin and Thiamine hydrochloride were freshly prepared as 0.06 g/L and 10 g/L stock solutions and sterile-filtered, respectively. The medium was prepared from the stock solutions and Kanamycin (Kanamycin solution, K0254, Sigma-Aldrich, St. Louis, MO, USA) was added for a final concentration of 50 mg/L.

For expression cultures sterilized 2.5-mL 96-well deep-well plates (850301, HJ-Bioanalytik, Erkelenz, Germany) were filled with 440 µL expression medium supplemented with kanamycin (50 mg/L); expression cultures were inoculated with 50 µL of pre-cultures. The expression culture plates were incubated at 37°C and 250 rpm in a climo shaker. After 4 h incubation 10 µL of 30 mM IPTG diluted in expression medium (supplemented with 50 mg/L kanamycin) was added per well (resulting in a final concentration of 0.6 mM IPTG). The expression culture plates were subsequently incubated for 20 hours at 27°C in a shaking incubator. Cells were harvested by centrifugation of the expression cultures for 15 minutes at 4°C and 2500 × g. The culture supernatant was discarded, and the remaining cell pellets were resuspended in 250 µL of 2x P450 assay buffer (200 mM KPO4-buffer at pH 7.0, 100 g/L glucose, 70 g/L glycerol, 2 mM EDTA). The deep-well plates were stored at -80°C until use.

### 2.2 Culture conditions at larger scale

For generating larger amounts of biomass *E. coli* strain BL 21 (DE3) was used as host for expression plasmids (Example 1). Pre cultures were inoculated from glycerol stocks and performed in shake flasks filled with LB-medium followed by a second pre culture in TB (Terrific Broth) media. The main cell culture was performed in DASGIP parallel bioreactor systems (Starting volume of 1 L). Medium for expression contained Yeast extract, ammonium phosphate, glycerol, Natrium chloride, 5-ALA. Induction was performed after 7,5 h with IPTG. pH was set to 6.6, temperature to 30 °C and stirrer to 500 rpm with 0,5 vvm air. Biomass was harvested after 24 h through centrifugation and used for subsequent biotransformation/ C19 hydroxylation.

### 2.3 Biotransformation/ C19 hydroxylation

### 2.3.1 Biotransformation/ C19 hydroxylation conditions in 96-well deep-well plates

Unless stated otherwise the following reaction conditions were used for the C19 hydroxylation (biotransformation) reaction in 96-well deep-well plates. The biotransformation media contained 50 g/L suspension of cells comprising the respective BM3 enzyme provided as a suspension in 2x P450 assay buffer that was previously frozen at -80°C,

The reaction was started by adding 250 µL per well of micronized androsta-1,4-diene-3,17-dione (ADD) suspension (at final concentration of 2 g/L).

Incubation occurred for 22 h at 27°C, 250 rpm in a Kühner shaker.

Reactions were stopped by adding 500 µL of Stop solution (containing Acetonitrile, Methanol and Dimethylsulfoxide in a 1:1:1 ratio), and for extraction of steroids incubated on a shaker for 30 min at 20°C. Afterwards the plates were centrifuged for 15 min at 4°C and 2500× g. From each well 120 µL of the supernatant was transferred to a 96-well filter plate (Corning, 3504) that was subsequently centrifuged for 5 min at 4°C and 650× g. The filtered samples were stored at -20°C or directly submitted to HPLC analysis.

### 2.3.2 Biotransformation/ C19 hydroxylation conditions at larger scale

Unless stated otherwise the following reaction conditions were used for the C19 hydroxylation. The biotransformation media contained 50 g/L suspension of cell comprising the respective BM3 enzyme provided either as fresh biomass directly after fermentation or as biomass previously frozen at -80°C, 0-25% (v/v) of fermentation-supernatant-media, 50 mM sodium phosphate buffer pH 6,6, 0-30 g/L Glucose-Monohydrate, 0,5 mM NADP, 0-1 g/L GDH-101 (from Johnson & Matthey), 0-300 mM K-formate, 0-5 mM GSH, 0-5 mM MgSO4, 0-0,5 g/L Lecithin and 0,1 ml/L Pluronic at pH 6,6.

The reaction was started by adding androsta-1,4-diene-3,17-dione (ADD) in a final concentration of 6-15 g/L either as micronized suspension or as powder or as solubilized solution in DMSO (final concentration of DMSO 1-5% (v/v)).

Biotransformation occurred for 20h-24h at 28°C. The media was stirred at 1300 rpm by using Rushton turbines. The pH was regulated to pH 6,6 by using 10% H2SO4 supplemented with 0-300 mM GSH (for reactions using FDH) or 3M potassium hydroxide (for reactions using GDH). A constant K-formate feed (1 g/h for 10 h of a 12 M K-formate solution) was applied at reactions with FDH or a constant D-glucose monohydrate feed (2,3 g/h for 7 h of a 500 g/L solution) when GDH-101 was used and the pO2 level was regulated at 30% by using pressured air.

Samples of the reaction were stopped by adding MIBK in a range of 1:1 - 1:3 (v/v), shaking at 2000 rpm for extraction and centrifuged at 16000× g for 2 minutes. From each sample 100 µL of the upper phase (=organic MIBK phase) was mixed with an internal standard (ca. 20 g/L Eicosan in MIBK) and analyzed per gas chromatography (GC). In case of reactions above 120 ml the device FlexyCube from System Technik Deutschland GmbH (Systag) was used.

### 2.4 Analytical GC method

### Gas chromatography device = Agilent GC 7890 A

- Column:: Agilent HP-5 50m, 320 µm x 0, 52µm
- Carrier gas:: helium
- Makeup gas:: helium
- Injector:: 250°C
- Split: 10:1
- Flow:: constant
- Detector:: FID 300°C (H2/air flow: 30/400ml/min)
- Injection volume:: 1 µl
- Run time:: 24,6 min

### 2.5 Analytical HPLC method

Analytical HPLC was performed with the following setup:
Instrument: Agilent Technologies 1290 Infinity; Column: Ascentis Express C18, 50 x 2.1mm, 2.7 µm; Eluent A: Water (+0.005% Trifluoroacetic acid); Eluent B: Acetonitrile; Flow 1.5 mL/min; Temperature: 45 °C; injection volume: 4 µL, detection at 280 nm. Gradient: 0 - 0.18 min 5% solvent B, 0.18 - 0.4 min 5 - 10% solvent B, 0.4 - 4.6 min 10 - 27% solvent B, 4.6 - 7.0 min 27 - 95% solvent B, 7.01 - 7.5 min 5% solvent B. Androsta-1,4-diene-3,17-dione (ADD) and estrone (dissolved in *N,N-*Dimethylformamide) were used as reference substances and as a standard for quantification. Appropriate dilutions were prepared covering the range up to the maximum concentration used in the biotransformation.

Observed retention times were 4.22 min for ADD and 4.86 min for estrone. The concentration of the respective compounds in samples was calculated by comparing the obtained peak areas with those of the standards.

### Example 3 Optimization of BM3 variants

Variants of SEQ ID NO 1 with mutations different positions were prepared as described in Example 1 and screened for androsta-1,4-diene-3,17-dione (ADD) to Estrone conversion. Cell culture biotransformation and HPLC analysis was performed as described in Example 2. Glycerol stocks were used as inoculum for cell cultivation, and the initial substrate androsta-1,4-diene-3,17-dione (ADD) concentration was 2000 mg/L.

Results are shown in Table 2 below. It can be seen that all variants comprising the mutations D80I, H236I and K440C achieved a better estrone titer i.e. an increased amount of steroid hydroxylated at C19 at 27°C than SEQ ID NO 1.

**Table 2**

| SEQ ID NO | Estrone @ 27°C Ratio-to-SEQ ID NO 1 |
|---|---|
| 2 | 14,314 |
| 3 | 6,361 |
| 4 | 14,692 |
| 5 | 2,466 |
| 6 | 10,813 |
| 7 | 10,706 |
| 8 | 16,132 |
| 9 | 6,876 |
| 10 | 6,437 |
| 11 | 4,576 |
| 12 | 10,21 |
| 13 | 2,698 |
| 14 | 2,583 |
| 15 | 4,95 |
| 16 | 2,689 |
| 17 | 10,828 |
| 18 | 3,914 |
| 19 | 11,348 |
| 20 | 3,571 |
| 21 | 9,323 |
| 22 | 11,83 |
| 23 | 5,092 |
| 24 | 6,097 |
| 25 | 9,204 |
| 26 | 18,928 |
| 27 | 15,431 |
| 28 | 3,497 |
| 29 | 6,322 |
| 30 | 9,872 |
| 31 | 3,942 |
| 32 | 5,18 |
| 33 | 5,295 |
| 34 | 3,392 |
| 35 | 10,501 |
| 36 | 16,471 |
| 37 | 2,707 |
| 38 | 18,093 |
| 39 | 3,407 |
| 40 | 3,677 |
| 41 | 4,284 |
| 42 | 2,697 |
| 43 | 3,541 |
| 44 | 3,423 |
| 45 | 12,966 |
| 46 | 2,181 |
| 47 | 4,615 |
| 48 | 9,119 |
| 49 | 2,717 |
| 50 | 6,053 |
| 51 | 5,048 |
| 52 | 5,358 |
| 53 | 2,411 |
| 54 | 1,879 |
| 55 | 1,795 |
| 56 | 11,141 |
| 57 | 2,159 |
| 58 | 4,137 |
| 59 | 5,425 |
| 60 | 4,780 |
| 61 | 8,140 |
| 62 | 7,699 |
| 63 | 3,689 |
| 64 | 2,126 |
| 65 | 8,009 |
| 66 | 2,391 |
| 67 | 2,729 |
| 68 | 7,602 |
| 69 | 4,607 |
| 70 | 3,242 |
| 71 | 4,158 |
| 72 | 2,741 |
| 73 | 2,827 |
| 74 | 7,241 |
| 75 | 2,256 |
| 78 | 1,452 |
| 79 | 0,964 |
| 80 | 0,751 |
| 81 | 1,407 |
| 82 | 1,293 |
| 83 | 1,280 |
| 84 | 0,459 |
| 85 | 0,609 |
| 86 | 0,843 |
| 87 | 0,816 |
| 88 | 0,648 |
| 89 | 0,695 |
| 90 | 0,099 |
| 91 | 0,946 |
| 92 | 0,864 |

The results of Table 2 demonstrated that surprisingly all BM3 monooxygenase (BM3) variants comprising - compared to SEQ ID NO 1 - at least the mutations D80I and H236I and K440C showed at least an 1.8 times improvement of estrone titer i.e. an increased amount of steroid hydroxylated at C19 compared to SEQ ID NO 1. In contrast other variants of SEQ ID NO 1 with only one or other mutations compared to SEQ ID NO 1 showed no or less improvement.

### Example 4 Optimization of C19 hydroxylation (Biotransformation reaction)

### Example 4.1 Effect of Freezing

The experiments were performed as described in section 2.3.2. The biotransformation media contained 50 g/L suspension of cell comprising the respective BM3 enzyme provided either as fresh biomass directly after fermentation or as biomass previously frozen at -80°C. 1 g/L GDH-101 was supplied.

The results are given in Table 3a below where for each tested enzyme the estrone titer (i.e. an increased amount of steroid hydroxylated at C19) without freezing is given the value 1 and the estrone titers obtained with previously frozen biomass are shown as multiples of this value.

**Table 3**

| Enzyme variant | frozen | fresh |
|---|---|---|
| SEQ ID NO 1 | 1,4 | 1 |
| SEQ ID NO 36 | 1 | 1 |
| SEQ ID NO 68 | 1,25 | 1 |

In Table 3b the multiples of the estrone titer of SEQ ID NO 1 without freezing are given.

**Table 3b**

| Enzyme variant | frozen | fresh |
|---|---|---|
| SEQ ID NO 1 | 4 | 3,2 |
| SEQ ID NO 36 | 3 | 3 |
| SEQ ID NO 68 | 1,25 | 1 |

The results demonstrated that freezing of the biomass containing the P540-BM3 enzyme prior to the biotransformation tended to increase the estrone titer compared to non-frozen biomass.

### Example 4.2 Effect of Toluene

The experiments were performed as described in section 2.3.2 with the following variations of the general conditions:. For the toluene treatment fresh biomass was resuspended into fermentation supernatant with a concentration of 200 g/L in 32 ml. Toluene was added to a final concentration of 1% (v/v). The Biomass was incubated for 20 min and then added into the biotransformation media. The same was done with the control reaction but without adding toluene. Furthermore, the same biomass was pelleted after fermentation by centrifugation and frozen at -30°C for comparing activity with frozen cells. 1 g/L GDH-101 was supplied. 3 g/L ADD were used.

It was shown that the use of frozen biomass resulted in a space time yield of 0,2 g/(L*d) the use of fresh biomass resulted in a space time yield of 0,05 g/(L*d) and the combination of fresh biomass and 1% toluene resulted in a space time yield of 0,2 g/(L*d). Thus, via treating the cells with 1% toluene the space-time yield of the enzyme provided in non-frozen cells was substantially increased to a level previously only achievable via freezing the cells, while being easier to scale-up to industrial scale .

### Example 4.3 Effect of GSH

The assay conditions were as follows:
Previously frozen biomass was tested in a screening approach for the effect of different GSH concentrations on product titer. For this 50 g/L wet frozen biomass harboring the BM3 variant was used in a reaction buffer with 6 g/L final concentration of ADD, 5% DMSO, 300 mM sodium phosphate buffer pH 7, 20 g/L glucose monohydrate, 0,5 mM NADP, 1 g/L GDH-101, 5 mM MgSO4 and 0-50 mM reduced GSH. The biotransformation was conducted in FlowerPlates from Beckmann Coulter and airpore sheets in a final reaction volume of 600 µl. The plates were shaken by a Thermoshaker C from Eppendorf at 1500 rpm, 28°C and for 22h.

The samples were prepared as followed: in each well 600 µl DMSO was added, carefully shaken to rinse clumps from well inner side for 10 min at 28°C. Extraction of product was realized by adding 600 µl MIBK (Methylisobutylketone) onto 600 µl sample and shaken at 30°C for 5 minutes followed by centrifugation 1 min centrifugation at 16.000 g. The upper MIBK phase was analyzed with the mentioned GC method.

The results are given in Table 4 below where for each tested enzyme the estrone titer without GSH addition is given the value 1 and the estrone titers with GSH addition are shown as multiples of this value (and in brackets the multiples of the estrone titer of SEQ ID NO 1 without GSH addition are given).

**Table 4**

| Enzyme variant | 0mM GSH | 5 mM GSH | 50 mM GSH |
|---|---|---|---|
| SEQ ID NO 1 | 1 | 1,3 | 0,8 |
| SEQ ID NO 36 | 1 (2,3) | 1,2 (2,1) | 0,9 (2,4) |
| SEQ ID NO 68 | 1 (3,5) | 1,3 (3,6) | 0,8 (3,4) |

The results demonstrate that addition of 5mM GSH increased the estrone titer.

### Example 4.4 Effect of FDH

### Example 4.4.1 Effect of FDH in combination with biomass previously frozen at -80°C

In this example the conditions as described under 2.3.2 were used.

The results demonstrated that the combination of freezing the cells prior to the biotransformation reaction and providing FDH increased the estrone titer by a factor of 3,9 compared to merely freezing the cells prior to the biotransformation reaction. A estrone titer of 7,7g/L was reached using the enzyme of SEQ ID NO 68 in this experiment.

### Example 4.4.2 Effect of FDH in combination with fresh biomass (non-frozen cells)

In this example the conditions as described under 2.3.2 were used.

The results demonstrated that providing FDH increased the estrone titer by a factor of 2,7 compared to the estrone titer of non-frozen cells. Moreover, the provision of FDH also increased the estrone titer of fresh biomass - i.e. cells that were not frozen prior to the biotransformation reaction - by a factor of 2,2 compared to cells which had been frozen prior to the biotransformation reaction, but to which no FDH was added. An estrone titer of 4,8/L was reached using the enzyme of SEQ ID NO 68 in this experiment.

### Example 4.5 Effect of GDH

In this example the conditions as described under 2.3.2 were used.

The results are given in Table 5a below where for each tested enzyme the estrone titer without GDH addition is given the value 1 and the estrone titers with GDH addition are shown as multiples of this value.

**Table 5a**

| Enzyme variant | GDH addition | w/o GDH addition |
|---|---|---|
| SEQ ID NO 1 | 1,5 (4,03) | 1 |
| SEQ ID NO 36 | 1,15 (2,4) | 1 |
| SEQ ID NO 68 | 1,01 | 1 |

In Table 5b the multiples of the estrone titer of SEQ ID NO 1 without GDH addition are given.

| Enzyme variant | GDH addition |
|---|---|
| SEQ ID NO 1 | 4,03 |
| SEQ ID NO 36 | 2,4 |
| SEQ ID NO 68 | 1,01 |

The results demonstrate that addition of GDH increased the estrone titer.

### Example 4.6 Effect of 5-Aminolevulinic acid ("5-Ala") addition

Biotransformation efficiencies of selected BM3 variants were tested with biomass from expression cultures supplemented with 0.5 mM 5-Aminolevulinic acid (5-Ala) in comparison to biomass from expression cultures without 5-Ala supplementation. Cell culture, biotransformation and HPLC analysis was performed as described in Example 2. Colonies from Agar plates served as inoculum for cell culture; and 0.5 mM 5-Ala was added to the expression culture upon induction of expression with IPTG for one set of samples, the other set (control) was induced with IPTG but without 5-Ala supplementation. The initial substrate androsta-1,4-diene-3,17-dione (ADD) concentration was 2000 mg/L.

When specifically indicated 10 µL of 30 mM IPTG and 25 mM 5-Aminolevulinic acid (5-Ala) diluted in expression medium were added per well to induce enzyme expression and to supplement precursor for heme biosynthesis (resulting in a final concentration of 0.6 mM IPTG and 0.5. mM 5-Ala). Results are shown in Table 6 below

**Table 6**

| SEQ ID NO | Ratio of estrone titer with 5-Aminolevulinic acid to Estrone titer without 5 Ala |
|---|---|
| 56 | 1,87 |
| 71 | 4,84 |
| 78 | 1,04 |
| 79 | 1,78 |
| 80 | 1,71 |
| 75 | 1,73 |
| 81 | 2,03 |
| 53 | 3,55 |
| 26 | 1,81 |
| 38 | 0,99 |
| 36 | 1,01 |
| 74 | 1,37 |
| 73 | 1,32 |
| 17 | 1,28 |
| 69 | 1,05 |
| 68 | 1,83 |
| 1 | 1,95 |

The results demonstrated that the addition of 5-Aminolevulinic acid increases the amount of steroid hydroxylated at C19.

For selected P450 BM3 monooxygenase (BM3) variants described herein the space time yield and the ratio of steroid to be hydroxylated at C19 to steroid hydroxylated at C19 were determined in comparison to WO2021156200 A1. The results are shown in Table 7 below:

**Table 7**

| | A | B |
|---|---|---|
| SEQ ID NO | g/l*d (22h) | 2g/L ADD : value of column A (g/L estrone (22h)) |
| 26 | 0,68 | 2,95 |
| 38 | 0,94 | 2,13 |
| 68 | 0,47 | 4,24 |
| 1 | 0,10 | 19,06 |

The results demonstrated that the addition of 5-Aminolevulinic acid resulted in a space time yield of more than 470 mg/l estrone per day and a ratio of steroid to be hydroxylated at C19 to steroid hydroxylated at C19 of less than 5 for the cytochrome P450 BM3 monooxygenase (BM3) variants described herein.

## Claims

1. A Cytochrome P450 BM3 monooxygenase (BM3) variant for catalyzing the C19 hydroxylation of a steroid or steroid derivative, wherein the BM3 variant comprises compared to SEQ ID NO 1 at least the mutations D80I and H236I and K440C.

2. A Cytochrome P450 BM3 monooxygenase (BM3) according to claim 1 wherein the BM3 variant has at least 99% sequence identity with SEQ ID NO 1 and comprises compared to SEQ ID NO 1 at least the mutations D80I and H236I and K440C.

3. The cytochrome P450 BM3 monooxygenase (BM3) variant of claim 1 or 2 wherein the BM3 variant comprises compared to SEQ ID NO 1 a combination of mutations selected from the list consisting of SEQ ID NO
2) Q73W, D80I, T175Y, A225V, H236I, G240R, K440C
3) Q73W, D80I, T175Y, A225V, H236I, K440C
4) Q73W, D80I, T175Y, D182H, H236I, G240R, K440C
5) Q73W, D80I, T175Y, D182H, A225V, H236I, G240R, K440C
6) Q73W, D80I, T175Y, D182H, A225V, H236I, K440C
7) Q73W, D80I, T175Y, D182H, H236I, K440C
8) Q73W, D80I, T175Y, D182I, H236I, G240R, K440C
9) Q73W, D80I, T175Y, D182I, A225V, H236I, G240R, K440C
10) Q73W, D80I, T175Y, D182I, A225V, H236I, K440C
11) Q73W, D80I, T175Y, D182I, H236I, K440C
12) Q73W, K76I, D80I, T175Y, H236I, G240R, K440C
13) Q73W, K76I, D80I, T175Y, A225V, H236I, G240R, K440C
14) Q73W, K76I, D80I, T175Y, A225V, H236I, K440C
15) Q73W, K76I, D80I, T175Y, D182H, A225V, H236I, G240R, K440C
16) Q73W, K76I, D80I, T175Y, D182I, H236I, G240R, K440C
17) Q73W, K76I, D80I, T175Y, D182I, A225V, H236I, G240R, K440C
18) Q73W, K76I, D80I, T175Y, R179L, H236I, G240R, K440C
19) Q73W, K76I, D80I, T175Y, R179L, D182I, H236I, G240R, K440C
20) Q73W, K76I, D80I, T175Y, H236I, K440C
21) Q73W, D80I, T175Y, R179L, H236I, G240R, K440C
22) Q73W, D80I, T175Y, R179L, A225V, H236I, G240R, K440C
23) Q73W, D80I, T175Y, R179L, D182H, H236I, G240R, K440C
24) Q73W, D80I, T175Y, R179L, D182H, A225V, H236I, G240R, K440C
25) Q73W, D80I, T175Y, R179L, D182H, H236I, K440C
26) Q73W, D80I, T175Y, R179L, D182I, H236I, G240R, K440C
27) Q73W, D80I, T175Y, R179L, D182I, A225V, H236I, G240R, K440C
28) Q73W, D80I, T175Y, R179L, D182I, A225V, H236I, K440C
29) Q73W, D80I, T175Y, R179L, D182I, H236I, K440C
30) Q73W, D80I, T175Y, R179L, H236I, K440C
31) Q73W, D80I, T175Y, H236I, K440C
32) Q73L, D80I, T175Y, H236I, G240R, K440C
33) Q73L, D80I, T175Y, A225V, H236I, G240R, K440C
34) Q73L, D80I, T175Y, A225V, H236I, K440C
35) Q73L, D80I, T175Y, D182H, H236I, G240R, K440C
36) Q73L, D80I, T175Y, D182I, H236I, G240R, K440C
37) Q73L, K76I, D80I, T175Y, H236I, G240R, K440C
38) Q73L, K76I, D80I, T175Y, A225V, H236I, G240R, K440C
39) Q73L, K76I, D80I, T175Y, R179L, H236I, G240R, K440C
40) Q73L, K76I, D80I, T175Y, R179L, A225V, H236I, G240R, K440C
41) Q73L, K76I, D80I, T175Y, R179L, D182H, A225V, H236I, G240R, K440C
42) Q73L, K76I, D80I, T175Y, H236I, K440C
43) Q73L, D80I, T175Y, R179L, H236I, G240R, K440C
44) Q73L, D80I, T175Y, R179L, A225V, H236I, K440C
45) Q73L, D80I, T175Y, R179L, D182I, A225V, H236I, K440C
46) Q73L, D80I, T175Y, R179L, D182I, H236I, K440C
47) Q73L, D80I, T175Y, R179L, H236I, K440C
48) Q73L, D80I, T175Y, H236I, K440C
49) D80I, F173W, H236I, G240R, T269V, K440C
50) D80I, F173W, T175Y, D182I, H236I, K440C
51) D80I, F173W, T175Y, H236I, K440C
52) D80I, F173W, T175Y, R179L, D182H, H236I, T269V, K440C
53) D80I, H236I, K440C
54) D80I, H236I, T269V, K440C
55) D80I, P172G, F173W, R179L, H236I, G240R, T269V, K440C
56) D80I, P172G, T175Y, R179L, D182I, H236I, G240R, K440C
57) D80I, R179L, D182H, H236I, T269V, K440C
58) D80I, R179L, D182I, H236I, T269V, K440C
59) D80I, R179L, H236I, K440C
60) D80I, T175Y, A225V, H236I, G240R, T269V, K440C
61) D80I, T175Y, H236I, G240R, K440C
62) K76I, D80I, F173W, T175Y, H236I, K440C
63) K76I, D80I, N186V, A225V, H236I, K440C
64) K76I, D80I, N186V, H236I, K440C
65) K76I, D80I, R179L, H236I, G240R, K440C
66) K76I, D80I, R179L, H236I, K440C
67) K76I, D80I, R179L, N186V, H236I, K440C
68) Q73W, D80I, T175Y, H236I, G240R, K440C
69) Q73W, K76I, D80I, T175Y, D182H, H236I, G240R, K440C
70) Q73W, K76I, D80I, T175Y, D182H, N186V, A225V, H236I, G240R, K440C
71) Q73W, K76I, D80I, T175Y, D182H, N186V, H236I, G240R, K440C
72) Q73W, K76I, D80I, T175Y, D182I, A225V, H236I, K440C
73) Q73W, K76I, D80I, T175Y, D182I, H236I, K440C
74) Q73W, K76I, D80I, T175Y, R179L, D182H, H236I, G240R, K440C
75) Q73L, D80I, T175Y, R179L, H236I, G240R, T269V, K440C.

4. The cytochrome P450 BM3 monooxygenase (BM3) variant of claim 1 to 3 wherein the BM3 variant comprises compared to SEQ ID NO 1 the further mutations Q73L, T175Y and G240R and is selected from the list consisting of
SEQ ID NO 38: Q73L, K76I, D80I, T175Y, A225V, H236I, G240R, K440C
SEQ ID NO 36: Q73L, D80I, T175Y, D182I, H236I, G240R, K440C
SEQ ID NO 35: Q73L, D80I, T175Y, D182H, H236I, G240R, K440C
SEQ ID NO 45: Q73L, D80I, T175Y, R179L, D182I, A225V, H236I, K440C
SEQ ID NO 48: Q73L, D80I, T175Y, H236I, K440C.

5. The cytochrome P450 BM3 monooxygenase (BM3) variant of claim 1 to 3 wherein the BM3 variant comprises compared to SEQ ID NO 1 the further mutations Q73W, T175Y and G240R and is selected from the list consisting of
SEQ ID NO 26: Q73W, D80I, T175Y, R179L, D182I, H236I, G240R, K440C
SEQ ID NO 74: Q73W, K76I, D80I, T175Y, R179L, D182H, H236I, G240R, K440C
SEQ ID NO 17: Q73W, K76I, D80I, T175Y, D182I, A225V, H236I, G240R, K440C
SEQ ID NO 68: Q73W, D80I, T175Y, H236I, G240R, K440C
SEQ ID NO 2: Q73W, D80I, T175Y, A225V, H236I, G240R, K440C
SEQ ID NO 4: Q73W, D80I, T175Y, D182H, H236I, G240R, K440C
SEQ ID NO 8: Q73W, D80I, T175Y, D182I, H236I, G240R, K440C
SEQ ID NO 12: Q73W, K76I, D80I, T175Y, H236I, G240R, K440C.

6. An in vitro method for generating a steroid hydroxylated at C19 using a cytochrome P450 BM3 monooxygenase (BM3) variant of claim 1 or claim 2 wherein the generated steroid hydroxylated at C19 is present in a range of 0,4 g/(L*d) to 50 g/(L*d).

7. An in vitro method according to claim 6 wherein the C19 hydroxylation is achieved in the same cells that generated the P450-monooxygenase.

8. An in vitro method according to claim 6wherein the ratio of steroid to be hydroxylated at C19 to steroid hydroxylated at C19 is in the range of between 1:4 to 1:1.

9. An in vitro method according to claim 6 wherein prior to C19 hydroxylation in said cells 5-Aminolevulinic acid is added to the cell culture medium.

10. An in vitro method according to claim 6 wherein prior to C19 hydroxylation in said cells toluene in the range of 0,2% (v/v) to 2 % (v/v) is added.

11. An in vitro method according to claim 6 wherein the C19 hydroxylation is carried out in the presence of FDH or GDH and/or wherein the C19 hydroxylation is carried out in the presence of GSH.

12. An in vitro method according to claim 6 further comprising following C19 hydroxylation purification of the steroid hydroxylated at C19.

13. A Cytochrome P450 BM3 monooxygenase (BM3) variant according to claim 1 wherein upstream of the enzyme sequence a His Tag is located.

14. A Cytochrome P450 BM3 monooxygenase (BM3) variant according to claims 1-4 or a method according to anyone of the preceding claims wherein the steroid to be hydroxylated at C19 is androsta-1,4-dien-3,17-dione (ADD).

15. An in vitro method according to claim 14 wherein the hydroxylation of androsta-1,4-dien-3,17-dione (ADD) at C19 leads to formation of estrone.
